(19) Europäisches Patentamt
European Patent Office
Office européen des brevets

(11) Publication number : **0 583 050 A2**

(12) # EUROPEAN PATENT APPLICATION

(21) Application number : **93303308.6**

(22) Date of filing : **28.04.93**

(51) Int. Cl.$^5$ : **C12N 15/12,** C07K 13/00, A61K 37/02, C12P 21/08, G01N 33/577, C12N 5/10, C12N 1/21, // (C12N1/21, C12R1:19)

(30) Priority : **29.04.92 US 877431**

(43) Date of publication of application : **16.02.94 Bulletin 94/07**

(84) Designated Contracting States : **AT BE CH DE DK ES FR GB GR IE IT LI LU MC NL PT SE**

(71) Applicant : **AMGEN INC.**
**1840 Dehavilland Drive**
**Thousand Oaks California 91320 -1789 (US)**

(71) Applicant : **YEDA RESEARCH AND DEVELOPMENT COMPANY, LTD.**
**Weizmann Institute of Science Herzl Street at Yavne Road P.O. Box 95**
**Rehovot 76100 (IL)**

(72) Inventor : **Wen, Duanzhi**
**517 Raindance Street**
**Thousand Oaks, California 91360 (US)**
Inventor : **Peles, Elior**
**209 Ben Yehuda Street**
**Tel Aviv 63502 (IL)**
Inventor : **Yarden, Yosef**
**7 Hachita Street**
**Rehovot (IL)**

(74) Representative : **Brown, John David et al**
**FORRESTER & BOEHMERT**
**Franz-Joseph-Strasse 38**
**D-80801 München (DE)**

(54) **Recombinant stimulating factor of the neu receptor.**

(57) Recombinant *neu* receptor stimulating factor, analogs of the factor, DNA sequences encoding the same, and methods of production are disclosed. Pharmaceutical compositions and methods of treating disorders involving *neu* receptor expression are also described.

FIG. 9

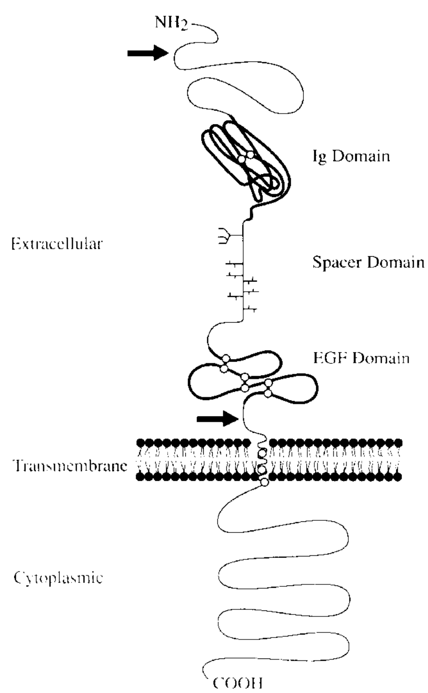


EP 0 583 050 A2

## Field Of The Invention

This invention relates to a novel non-naturally-occurring polypeptide, produced by recombinant DNA methods, which interacts with and stimulates the *neu* receptor and modulates cellular proliferation and differentiation. The invention also relates to analogs and derivatives of the polypeptide, as well as to DNA sequences encoding the polypeptide, analogs and derivatives.

## Background Of The Invention

Cell growth and differentiation are regulated in part by extracellular signals that are mediated by polypeptide molecules (Aaronson, S.A., *Science 254*:1146-1152, 1991). The interaction between these factors and specific cell surface receptors initiates a biochemical cascade culminating in nuclear events that regulate gene expression and DNA replication (Ullrich, A. and Schlessinger, J., *Cell 61*:203-212, 1990). This mechanism of cell regulation has implications for the determination of cell fate during development and breakdown of this mechanism may lead to oncogenic transformation. The latter can be induced by constitutive production of growth regulatory factors or by altered forms of their cognate receptors (Yarden, Y., and Ullrich, A., *Ann. Rev. Biochem. 57*:443-448, 1988). The oncogenic receptors belong to a family of transmembrane glycoproteins that share a common catalytic function in their cytoplasmic domains, namely, a tyrosine-specific protein kinase activity (Hanks, S.K., *Cur. Op. Struct. Biol. 1*:364-383, 1991) . The *neu* proto-oncogene (also called *erb*B-2 and HER-2) encodes a tyrosine kinase that is highly related to the receptor for the epidermal growth factor (King, C.R. et al., *EMBO J. 7*:1647-1651, 1988; Coussens, L. et al., *Science 230*:1132-1139, 1985; Bargmann, C.I., et al., *Cell 45*:649-657, 1986; Yamamoto, T. et al., *Nature 319*:230-234, 1986). Both the *neu* receptor and the EGF-receptor share a cysteine-rich structure at their extracellular domains that is connected through a single transmembrane stretch of amino acids to a large cytoplasmic domain having the enzymatic function. The oncogenic potential of the *neu* receptor can be released by multiple genetic mechanisms, including a point-mutation within the transmembrane region (Bargmann et al., *supra)* and truncations of non-catalytic sequences at both the cytoplasmic and the extracellular domains (DiFiore, P.P. et al., *Science 237*:178-182, 1987; Bargmann, C.I., and Weinberg, R.A., *EMBO J. 7*:2043-2052, 1988).

A third mechanism of oncogenic activation which has been implicated in the pathogenesis of human cancer involves overexpression of the apparently normal gene. Amplification and overexpression of the *neu* receptor have been detected with great frequency in human adenocarcinomas from several tissues (Kraus. M.H. et al., *EMBO J. 6*:605-610, 1987; Slamon, D.J. et al., *Science 235*:177-182, 1987; Varley, J.M. et al., *Oncogene 1*:423-430, 1987; van de Vijver, M. et al., *Mol. Cell Biol. 7*:2019-2023, 1987). Moreover, an association between gene amplification and overexpression and clinical outcome has been reported in breast and ovarian cancer (Slamon et al., *supra;* Varley et al., *supra;* Venter, D.J. et al., *Lancet ii*:67-72, 1987; Zhou, D. et al., *Cancer Res. 47*:6123-6125, 1987; Berger, M.S., et al., *Cancer Res. 48*:1238-1243, 1988; Tsuda, H. et al., *Cancer Res. 49*:3104-3108, 1989; Slamon, D.J., et al., *Science 244*:707-712, 1989). Consistent with the possibility that the neu receptor is involved in these malignancies, the overexpression of this receptor in an experimental model leads to the appearance of a transformed phenotype (DiFiore et al., *supra;* Hudziak, R.M. et al., *Proc. Natl. Acad. Sci. USA 84*:7159-7163, 1987). The mechanism responsible for the transforming potential of an overexpressed neu protein is not known, but it may involve constitutive activity of the intrinsic tyrosine kinase in the absence of ligand (Lonardo, F. et al., *New Biol. 2*:992-1003, 1990).

Simultaneous overexpression of the *neu* receptor and the EGF-receptor synergistically transforms rodent fibroblasts (Kokai, Y. et al., *Cell 58*:287-292, 1989) and is observed in human cancers (Harris, A.L. et al., *Molecular Diagnostics of Human Cancer,* Volume 7, edited by Furth, M., and Greaves, M., Cold Spring Harbor Press, New York, 1989; Gullick, W.J., *Int. J. Cancer.*Suppl. 5, pp. 55-61, 1990). The collaboration between these two receptors is probably mediated by a transregulatory effect of the EGF-receptor on the *neu* receptor, involving an increased tyrosine phosphorylation of the latter (Stern, D.J., and Kamps, M.P. *EMBO J. 7*:995-1001, 1988; King, C.R. et al., *EMBO J. 7*:1647-1651, 1988; Kokai, Y. et al., *Proc. Natl. Acad. Sci. USA 85*:5389-5393, 1988). Mechanistically, these interactions are mediated by heterodimerization of the EGF-receptor and the *neu* receptor (Goldman, R. et al., *Biochemistry 29*:11024-11028, 1990; Wada T. et al., *Cell 61*:1339-1347, 1990). These observations raise the possibility that the *neu* receptor may be regulated by other receptor tyrosine kinases that belong to the EGF-receptor sub-family, for example, the *erb*B-3 protein (Kraus, M.H. et al., *Proc. Natl. Acad. Sci.USA 86*:9193-9197, 1989; Plowman, G.D. et al., *Proc. Natl. Acad . Sci. 87*:4905-4909, 1990).

In contrast to the EGF-receptor, which is known to have multiple ligand molecules sharing an EGF-like motif (Carpenter, G., and Cohen, S., *J. Biol. Chem. 265*:7709-7712, 1990), a ligand that directly interacts with the *neu* receptor has not been completely characterized before. The existence of a candidate *neu* ligand in the culture medium of *ras*-transformed fibroblasts has been reported (Yarden, Y., and Weinberg, R.A., *Proc. Natl. Acad. Sci. USA 86:*3179-3188, 1989). The factor was partially purified from this source based on its ability to increase the phosphorylation of the *neu* receptor on tyrosine residues (Yarden, Y., and Peles, E., *Biochemistry 30*:3543-3550, 1991). This activity corresponds to a heat-stable and disulfide-containing glycoprotein that chromatographically behaved as a 30-35 kilodalton protein. By using different ligand assays, a 30-kilodalton glycoprotein has been isolated from the culture medium of the MDA-MB-231 human breast carcinoma cells (Lupu, R. et al., *Science 249*:1552-1555, 1990), and another factor was partially purified from the culture medium of transformed human T cells (Dobashi, K. et al., *Proc. Natl. Acad. Sci. USA 88*:8582-8586, 1991).

Recently, a factor which stimulates and apparently binds to the *neu* receptor has been purified to homogeneity from the conditioned medium of *ras*-transformed rat fibroblasts. When tested on human breast cancer cells, the factor, which is an approximately 44 kilodalton protein, was able to induce differentiation into mature milk-secreting cells (Peles, E. et al., *Cell 69*:205-216, 1992).

## Summary of the Invention

According to the present invention, a novel non-naturally-occurring neu receptor stimulating factor and isolated DNA sequences encoding all or part of the factor are provided. The factor of this invention is a polypeptide having an amino acid sequence sufficiently duplicative of that of naturally-occurring *neu* receptor stimulating factor (i.e., the polypeptides described by Peles, E. et al., in *Cell 69*: 205-216, 1992) to allow possession of one or more biological activities of the naturally-occurring factor (e.g., human *neu* receptor stimulating activity, human mammary tumor cell differentiating activity, ability to compete with naturally-occurring *neu* receptor stimulating factor for human receptor binding, etc.).

The *neu* receptor stimulating factor (also referred to herein as "NRSF") purified from natural sources has the ability to stimulate tyrosine phosphorylation of the polypeptide product of the human *neu* proto-oncogene and to induce differentiation of human mammary tumor cells to milk-producing, growth-arrested cells (Peles E. et al., *Cell, supra).* The biological activities of NRSF demonstrate its role as a human cell growth and differentiation factor and its potential application in a variety of therapeutic settings. The non-naturally-occurring *neu* receptor stimulating factor of the present invention will be useful, alone or in combination with other therapy, for the same human disorders as naturally-occurring *neu* receptor stimulating factor.

The isolated DNA sequences provided by the present invention are useful in securing expression in procaryotic and/or eucaryotic host cells of the non-naturally-occurring *neu* receptor stimulating factor of this invention (i.e., "recombinant NRSF"). The present invention specifically provides DNA sequences encoding the unprocessed amino acid sequence as well as DNA sequences encoding the processed (mature) forms of NRSF. Such DNA sequences include:

(a) the DNA sequences set out in Figures 4 and 5 (SEQ ID NO:3 and SEQ ID NO:4, respectively) and their complementary strands;

(b) DNA sequences which hybridize to the DNA sequences defined in (a) or fragments thereof; and

(c) DNA sequences which, but for the degeneracy of the genetic code, would hybridize to the DNA sequences defined in (a) and (b).

Specifically included in parts (b) and (c) are cDNA and genomic DNA sequences encoding variant forms of NRSF (including NRSF from other mammalian species), and manufactured DNA sequences encoding NRSF, fragments of NRSF, and analogs of NRSF. The DNA sequences may incorporate codons facilitating transcription and translation of messenger RNA in host cells. Manufactured sequences may readily be constructed according to the methods of Alton et al., PCT published application WO 83/04053.

Also provided are vectors containing such DNA sequences, and host cells transformed or transfected with such vectors. Additionally, the invention includes methods of producing NRSF by recombinant DNA techniques, and methods of treating disorders using recombinant NRSF. Pharmaceutical compositions containing recombinant NRSF and antibodies generated with recombinant NRSF are also provided.

## Brief Description Of The Drawings

Figure 1 depicts a high pressure liquid chromatogram of the trypsin digest of naturally-occurring *neu* receptor stimulating factor purified from media conditioned by ras-transformed rat fibroblasts according to the methods of Peles et al., *Cell, supra.* The elution profile is shown, and the amino acid sequences obtained from the fractions corresponding to each peak are indicated in conventional single letter designation. The amino acid in parenthesis represents an asparagine-linked glycosylation site, while the dotted line represents a longer sequence in which the amino acids were not identified. The inset shows re-chromatographic separation of the 27.3-minute peak after dithiothreitol reduction.

Figure 2 shows the structure of mammalian COS-7 cell expression vector pJT-2/NRSF. The Rat NRSF cDNA insert is the clone 44 cDNA sequence of Figure 5 (SEQ ID NO:4).

Figure 3 demonstrates stimulation of human *neu* receptor tyrosine phosphorylation by recombinant NRSF. Human MDA-MB-453 breast carcinoma cells were incubated with concentrated conditioned media from COS-7 monkey cells transfected with the indicated partially purified cDNA clones (left panel) or with fully purified cDNA clones (right panel). Positive controls comprised 10 ng/ml (left lane) and 100 ng/ml of purified naturally-occurring rat NRSF. Negative controls comprised concentrated media conditioned by untransfected COS-7 cells (lane designated "COS"), or by cells transfected with pJT-2 plasmids containing unrelated cDNA (lanes marked as "clone 27" and "clone 29"), and no addition ("NONE"). Lysates were prepared from the stimulated MDA-MB-453 cells and subjected to SDS-PAGE. Shown are autoradiograms of anti-phosphotyrosine Western blots, with the locations of molecular weight marker proteins given in kilodaltons (kDa). The following volumes of the COS-7 supernatants were used in a total volume of 0.125 ml PBS containing 0.1% BSA: 0.01 ml (left lane) and 0.1 ml (right lane) for clones 4, 19 and 44. The assay of the purified clones was performed in 0.25 ml total volume with 0.2 ml of cell supernatants or as indicated.

Figure 4 (SEQ ID NO:3) shows the nucleotide sequence of rat NRSF cDNA and a deduced amino acid sequence. Depicted is the combined nucleotide sequence of four cDNA clones. The beginning of the clone 44 DNA sequence in particular is indicated by an arrow. Nucleotide numbers and amino acid numbers are given in the left and right columns, respectively. Potential sites of N-linked glycoprotein are marked by asterisks, and cysteine residues found in the presumed extracellular domain are encircled. The overlining indicates peptide sequences that were determined directly from purified naturally-occurring NRSF. The underlining indicates the potential transmembrane region, whereas the dashed underlining indicates the polyadenylation sites. The portions of the protein sequence that contain an immunoglobulin (Ig) homology unit and an epidermal growth factor (EGF)-like motif are indicated on the right hand side.

Figure 5 (SEQ ID NO:4) shows the nucleotide sequence of NRSF cDNA clone 44 separately and a deduced amino acid sequence. Nucleotide numbers are given in the right hand column.

Figure 6 depicts the hydropathy profile of the precursor of the rat neu receptor stimulating factor. The method of Kyte and Doolittle, *J. Mol. Biol.* *157*:105-132 (1982), was used with a window size of nine amino acid residues. Positive values indicate increasing hydrophobicity. Amino acid numbers are given below the profile.

Figure 7 shows the alignment of the amino acid sequence of the EGF-like domain and the flanking carboxy terminal sequence of rat NRSF (SEQ ID NO:5) with representative members of the EGF family. Alignment and numbering begin at the most amino terminal cysteine residue of the EGF motifs. Amino acid residues are indicated by the single letter code. Dashes indicate gaps that were introduced for maximal alignment. Residues are boxed to indicate their identity with the corresponding amino acids of NRSF. The underlines indicate the amino terminal portions of the putative transmembrane domains that flank some of the EGF motifs at their carboxy terminal side. Asterisks show the carboxy termini. The following proteins are compared to NRSF: rat transforming growth factor $\alpha$ (TGF$\alpha$(SEQ ID NO:6); Marquardt, H. et al., *Science 223*:1079-1082, 1984), human amphiregulin (AR (SEQ ID NO:7); Shoyab, M. et al., *Science 243*:1074-1076, 1989), sheep fibroma virus growth factor (SFGF (SEQ ID NO:8); Chang, W. et al., *Mol. Cell. Biol. 7*:535-540 1987); myxoma virus growth factor (MGF (SEQ ID NO:9); Upton, C. et al., *J. Virol. 61*:1271-1275, 1987; mouse EGF (SEQ ID NO:10) (Gray, A. et al., *Nature 303*:722-725, 1983; Scott, J. et al., *Science 221*:236-240, 1983); human heparin-binding EGF (HB-EGF (SEQ ID NO:11); Higashiyama, S. et al., *Science 251*: 936-939, 1991); rat schwannoma-derived growth factor (SDGF (SEQ ID NO:12); Kimura, H. et al., *Nature 348*:257-260, 1990); and vaccinia virus growth factor (VGF (SEQ ID NO:13); Blomquist, M.D. et al., *Proc. Natl. Acad. Sci.* USA *81*:7363-7367, 1984).

Figure 8 shows the alignment of the immunoglobulin (Ig)-like domain of rat NRSF (SEQ ID NO:14) with the fourth Ig-related sequence of the murine neural cell adhesion molecule (SEQ ID NO:15) (NCAM; Barthels, D. et al., EMBO J. *6*: 907-914, 1987), a representative protein of the C2-set of the immunoglobulin superfamily. Residues that are highly conserved in the C2-set are indicated on the lower line, and positions conserved across the superfamily (according to Williams, A., and Barclay, A., *Rev. Immunol. 6*:381-405, 1988) are overlined. Stretches of amino acids that may be involved in $\beta$-strand formation are heavily underlined and labeled B through F, in analogy with the immunoglobulin domains. Boxes indicate identical residues in NRSF and NCAM. Dashes (gaps) were introduced for maximal alignment.

Figure 9 is a schematic presentation of the presumed secondary structure and membrane orientation of the precursor of the *neu* receptor simulating factor. Positions corresponding to the immunoglobulin (Ig)-domain and epidermal growth factor (EGF) motif are shown by thick lines and their cysteine residues are indicated by circles. The disulfide linkage of the Ig domain was directly demonstrated by amino acid sequence analysis (Example 1D), and the secondary structure of the EGF-domain is based on the homology with the EGF family. Also shown are the three cysteine residues found in the transmembrane domain. Arrows mark the processing sites of the precursor protein at the amino terminus (based on N-terminal sequencing of rat NRSF protein) and a putative proteolytic site close to the plasma membrane. The branched line indicates the location of a proven N-glycosylation site and the short vertical lines represent presumed sites of O-glycosylation.

Figure 10 depicts a receptor competition assay using radiolabeled naturally-occurring *neu* receptor stimulating factor purified from ras-transformed rat fibroblast conditioned media ("$^{125}$I-NRSF") . The labelled NRSF was incubated with human MDA-MB-453 breast carcinoma cells in the absence ("NONE") or presence of conditioned media from COS-7 cells expressing either recombinant *neu* receptor stimulating factor ("C-NRSF") or recombinant TGF$\alpha$ ("C-TGF$\alpha$"). The cell-bound radioactivity is shown as average $\pm$ S.D. (n=3).

Figure 11 depicts another receptor competition assay. $^{125}$I-labeled naturally-occurring NRSF was incubated with human MDA-MB-453 cells in the absence ("NONE") or presence of unlabeled "cold" naturally-occurring NRSF ("NRSF"; 200 ng/ml), or media conditioned by COS-7 cells that were transfected either with the NRSF expression plasmid of Fig. 2 ("C-NRSF") or with a TGF$\alpha$-encoding vector ("C-TGF$\alpha$"). Following crosslinking with BS$^3$, the cells were lysed and the *neu* receptor protein was immunoprecipitated by using a monoclonal antibody. Shown is an autoradiogram (3-day exposure) of the polyacrylamide gel-separated immunocomplexes. Molecular weights of marker proteins are indicated in kilodaltons. Mostly the presumed receptor dimer was radiolabeled.

Figure 12 shows autoradiograms obtained from Northern blots of mRNA isolated from cultured cells (panels A and C) or freshly isolated tissue of an adult rat (panel B), using clone 44 NRSF cDNA as a probe. The autoradiograms were obtained after exposure to film for 3 hours (panel A) or twenty-four hours (panels B and C). Molecular weights are shown in kilobases. Molecular weight estimation was performed by using a mixture of marker molecules from GIBCO BRL Life Technologies, Inc. (Gaithersburg, MD).

## Detailed Description Of The Invention

The DNA sequences of this invention are valuable as products useful in effecting the large scale synthesis of NRSF by a variety of recombinant techniques. Put another way, DNA sequences provided by the invention are useful in generating new and useful viral and circular plasmid DNA vectors, new and useful transformed and transfected procaryotic and eucaryotic host cells (including bacterial and yeast cells and mammalian cells grown in culture), and new and useful methods for cultured growth of such host cells capable of the expression of NRSF and its related products.

DNA sequences of the invention are also suitable materials for use as probes in isolating human cDNA and genomic DNA encoding NRSF and other genes encoding related proteins, including cDNA and genomic DNA sequences of other mammalian species. DNA sequences may also be useful in various alternative methods of protein synthesis (e.g., in insect host cells) or in genetic therapy in humans and other mammals. DNA sequences of the invention are expected to be useful in developing transgenic mammalian species which may serve as eucaryotic "hosts" for production of NRSF and NRSF products in quantity. See, generally, Palmiter et al., *Science 222,* 809-814 (1983). Diagnostic applications of NRSF DNA sequences of this invention are also possible, such as for the detection of alterations of genes and of mRNA structure and expression levels.

The recombinant NRSF of this invention is characterized by being the product of procaryotic or eucaryotic host expression (e.g., by bacterial, yeast, higher plant, insect or mammalian cells in culture) of exogenous DNA sequences obtained by genomic or cDNA cloning or by gene synthesis, particularly with use of the exogenous DNA sequence carried on an autonomously replicating DNA plasmid or viral vector in accordance with conventional techniques. The product of expression in typical yeast (e.g., *Saccharomyces cerevisiae)* or procaryote (e.g., *E. coli*) host cells is free of association with any mammalian proteins. The product of expression in vertebrate [e.g., non-human mammalian (e.g. COS or CHO) and avian] cells is free of association with any human proteins. Depending upon the host employed, the recombinant NRSF of the invention may be glycosylated with mammalian or other eucaryotic carbohydrates or may be non-glycosylated. The recombinant NRSF of the invention may also include an initial methionine amino acid residue (at position -1).

The present invention also embraces products such as polypeptide analogs of NRSF. Such analogs include fragments of NRSF. Following known procedures, one can readily design and manufacture genes encoding related polypeptides having primary structures which differ from that herein specified for in terms of the identity or location of one or more residues (e.g., substitutions, terminal and intermediate additions and deletions). Alternately, modifications of cDNA and genomic nucleotide sequences can be readily accomplished by well-known site-directed mutagenesis techniques and employed to generate analogs and derivatives of NRSF. Such products share one or more of the biological properties of naturally-occurring NRSF but may differ in others. As examples, products of the invention include those which are foreshortened by, e.g., deletions; or those which are more stable to hydrolysis (and, therefore, may have more pronounced or longer-lasting effects than naturally-occurring NRSF); or which have been altered to delete one or more potential sites for O-glycosylation and/or N-glycosylation or which have one or more cysteine residues deleted or replaced by other residues, e.g., alanine or serine residues, and are potentially more easily isolated in active form from microbial systems; or which have one or more tyrosine residues replaced by phenylalanine and bind more or less readily to target proteins or to receptors on target cells. Also included are polypeptide fragments duplicating only a part of the continuous amino acid sequence or secondary conformations within NRSF, which fragments may possess one property of NRSF and not others. It is noteworthy that the described activities are not necessary for any one or more of the products of the invention to have therapeutic utility or utility in other contexts, such as in NRSF antagonism. Competitive antagonists may be quite useful in, for example, cases of overproduction of NRSF.

The present invention also includes that class of polypeptides encoded by portions of the DNA complementary to the protein-coding strand of the human cDNA or genomic DNA sequences of NRSF.

Also encompassed by the invention are pharmaceutical compositions useful in treating biological disorders associated with neu receptor expression, comprising therapeutically effective amounts of polypeptide products of the invention together with suitable diluents, preservatives, solubilizers, emulsifiers, adjuvants and/or carriers useful in recombinant NRSF therapy. A "therapeutically effective amount" as used herein refers to that amount which provides therapeutic effect for a given condition and administration regiment. Such compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimerosol, benzyl alcohol) and bulking substances (e.g., lactose, mannitol); covalent attachment of polymers to the polypeptide to prolong *in vivo* half-life and to enhance potency (e.g., water-soluble polymers as polyethylene glycol and copolymers of polyethylene glycol and polypropylene glycol, see Davis et al., U.S. Patent No. 4,179,337); incorporation of the material into particulate preparations of polymeric compounds such as polylactic acid, polyglycolic acid, etc. or into liposomes. Such compositions will influence the physical state, stability, rate of *in vivo* release, and rate of *in vivo* clearance of recombinant NRSF.

The non-naturally-occurring *neu* receptor stimulating factor of this invention is expected to be useful, alone or in combination with other therapy, in treating diseases and conditions involving cells which express the *neu* receptor on their surfaces. In particular, the recombinant NRSFs of this invention are useful as growth inhibitor and differentiation factors for certain human carcinoma cells and will be applicable to the therapeutic treatment of various types of tumors related to *neu* receptor expression, including breast, ovarian, prostate and gastric carcinomas.

The factor may also be combined with substances such as radiolabeled molecules, toxins, cytokines, and other compounds useful in tumor treatment, in order to increase localization of these compounds on human tumors expressing high levels of the *neu* receptor.

Other biological or therapeutic applications are also possible. For example, normal human epithelial cells of the gastrointestinal, respiratory, urinary and reproductive tracts, as well as the skin, express the *neu* receptor on their surfaces (Press et al., *Oncogene 5*:953-962, 1990). Substances which bind to, or interact with, this receptor and alter cell growth or metabolism would be useful in situations where cell repopulation is needed, i.e., after physical injury resulting in cellular destruction, or where it is desired to increase or stimulate metabolic activities of the cell or properties resulting from cell growth and differentiation, e.g., renewed hair growth from follicles of the skin.

The polypeptides of the invention will be formulated and dosed according to the specific disorder to be treated, the condition of the individual patient, the site of delivery of the factor, the method of administration, and other circumstances known to practitioners. Thus, for the purposes herein, an effective amount of recombinant NRSF or analog or derivative is an amount that is effective to alter cellular proliferation and differentiation, or to prevent, lessen the worsening of, alleviate or cure the condition for which the polypeptide is administered. The activity of the present polypeptide may be enhanced or supplemented with use of one or more additional biologically active agents which are known to be useful in treating the same condition for which the polypeptide of the invention is being administered, e.g., IL-2 for cancer therapy, platelet-derived growth factors, epidermal growth factor, fibroblast growth factors, and the like for wound healing, and so forth.

## Description of the Specific Embodiments

The invention is illustrated in the following examples, which are not intended to be limiting. Biological materials employed in these examples were obtained as follows. The monoclonal antibody (Ab-3) to the carboxy terminus of the *neu* receptor was from Oncogene Science (Uniondale, NY). A monoclonal antibody to phosphotyrosine, PY20, was obtained from Amersham (Arlington Heights, IL). A mouse monoclonal antibody to human casein (types β and κ) was a gift from R.C. Coombs (Charing Cross Medical School, London). AU-565 human breast carcinoma cells were obtained from the Cell Culture Laboratory, Naval Supply Center (Oakland, CA). The Ratl-EJ cell line (ATCC CRL 10984) was generated by transfection of the human EJ *ras* oncogene into Rat1 fibroblasts as described by Peles, E. et al. in *Cell 69*:205-216, 1992 and by Land, H. et al., in *Nature 304*:596-602, 1983. Cell lines obtained from the American Type Culture Collection (Rockville, MD) included: MDA-MB-231 (ATCC HTB 26), MDA-MB-453 (ATCC HTB 131), Hs 294T (ATCC HTB 140), SK-BR-3 (ATCC HTB 30), HT-1080 (ATCC CCL 121), BALB/c 3T3 (ATCC CRL 6587) and COS-7 (ATCC CRL 1651). Cells were cultured as recommended by the American Type Culture Collection, or in Dulbecco's modified Eagle medium (GIBCO, Grand Island, NY) supplemented with 10% fetal bovine serum (Hyclone, Logan, Utah).

EXAMPLE 1

Amino Acid Sequence Analysis of Naturally-Occurring Rat NRSF

A. Tryptic Digestion

The purification and amino-terminal sequencing of the approximately 44-kilodalton naturally-occurring NFSF glycoprotein from media conditioned by *ras*-transformed rat fibroblasts (Rat 1-EJ cells) has been described in Peles, E. et al., *Cell 69*:205-216 (1992). In order to obtain more amino acid sequence information for the design of independent oligonucleotide probes, 300 picomoles of the purified rat protein were subjected to partial proteolysis with trypsin, as follows. Ten micrograms of the protein were reconstituted in 200 μl of 0.1 M ammonium bicarbonate buffer (pH 7.8). Digestion was conducted with L-1-tosylamido-2-phenylethyl chloromethyl ketone-treated trypsin (Serva) at 37°C for eighteen hours, using an enzyme-to-substrate ratio of 1:10.

B. Separation of Tryptic Digests by HPLC

The resulting peptide mixture was separated by reverse-phase HPLC and monitored at 215 nm using a Vydac C4 micro column (2.1 mm i.d. x 15 cm, 300 Å) and an HP 1090 liquid chromatographic system equipped with a diode-array detector and a workstation (Fig. 1). The column was equilibrated with 0.1% trifluoroacetic acid (mobile phase A) and elution was affected with a linear gradient from 0-55% mobile phase B (90% acetonitrile in 0.1% trifluoroacetic acid) over seventy minutes. The flow rate was 0.2 ml/min and the column temperature was controlled at 25°C. Three absorbance peaks eluted from the column very early (retention time less than five minutes), suggesting that these fractions correspond to short-length peptides. Three other, major fractions were recovered for amino acid sequence determination: a first fraction eluted after thirteen minutes (T13.3), a second after twenty-one minutes (T21.8), and a third after twenty-seven minutes (T27.3).

C. Sequencing of Eluted Peptide Fractions

The amino acid sequences of the peptides corresponding to the three major eluted fractions were determined by automated Edman degradation. Amino acid sequence analyses of the peptides were performed with a Model 477 protein sequencer (Applied Biosystems, Inc., Foster City, CA) equipped with an on-line phenylthiohydantoinyl (PTH) amino acid analyzer and a Model 900 data analysis system (Hunkapiller, M.W. et al., *Methods of Protein Microcharacterization,* Humana Press, Clifton, NJ, pages 223-247, 1986). The protein was loaded onto a trifluoroacetic acid-treated glass fiber disc precycled with polybrene and NaCl. The PTH-amino acid analysis was performed with a micro liquid chromatography system (Model 120) using dual syringe pumps and reverse-phase (C-18) narrow bore columns (Applied Biosystems, 2.1 mm x 250 mm). The first fraction (T13.3) yielded two distinguishable major sequence signals (signal ratio 5:1) with the length of three and four amino acids (the sequences are indicated in Fig. 1). The second fraction (T21.8) gave a single sequence, starting at residue number 9 of the previously determined primary N-terminal amino acid sequence (Peles et al., *supra;* the eighth residue is arginine). The sequence of peptide T21.8 thus confirmed the information obtained from the N-terminal sequence analysis of the whole protein and also assigned an aspartate to position 17, that was previously reported as an unassigned residue. The third fraction (T27.3) gave three distinct sequencing signals up to cycle 12 of Edman sequencing, and one clear sequence from cycle 13 through 24, suggesting that this third peptide is even longer.

D. Re-Chromatographing and Sequencing of Co-Eluted Peptides.

To precisely determine the amino acid sequences of the co-eluting peptides in fraction T27.3, an aliquot of that fraction was treated as follows. A seventy percent aliquot of the peptide fraction was dried *in vacuo* and reconstituted in 100 μl of 0.2 M ammonium bicarbonate buffer (pH 7.8). Dithiothreitol (final concentration 2 mM) was added to the solution which was then incubated at 37°C for thirty minutes. The reduced peptide mixture was then separated by reverse-phase HPLC using a Vydac column (2.1 mm i.d. x 15 cm). Elution conditions and flow rate were identical to those described previously.

Two major peptide peaks were recovered and sequenced by automated Edman degradation as described above, namely T34.4, an 11-residue arginine peptide and T40.4, a 12-amino acid long lysine peptide, (Fig. 1, inset). Residue 1 of peptide T34.4 and cycle 11 from T40.4 remained unassigned, suggesting that they may be cysteine residues related to the disulfide bond of peptides in fraction T27.3. This possibility was confirmed by electro-spray mass spectrometric analysis using an API-III mass spectrometer (SCIEX, Toronto, Canada). Both peptides T34.4 and T40.4 were analyzed, resulting in average mass figures of 1261.5 and 1274.0, respectively. It was therefore concluded that these two peptides are held together in the naturally-occurring protein by a disulfide linkage. On the basis of these data, the mixed sequence signals of peak T27.3 could be re-examined. When the sequences of peptides T34.4 and T40.4 were subtracted from the mixed signals of fraction T27.3, the sequence of the longer third peptide became apparent also for the first 12 cycles. The deduced 24-amino acid long sequence of this peptide is indicated in Figure 1. The 9th amino acid in this peptide sequence was assigned as an asparagine but at a much lower yield (about 10% of the sequence signal), suggesting a site for N-linked glycosylation.

In view of the molecular weight of naturally-occurring NRSF and the estimation that approximately one-quarter of its molecular mass is contributed by sugar moieties (Peles, E. et al., *Cell* 69:205-216, 1992), it was unexpected that only a few peptides were recovered after partial proteolysis. One possibility is that the naturally-occurring protein contains a protease-resistance core region that remained intact during proteolysis, but underwent denaturation and therefore was not resolved by HPLC chromatography.

EXAMPLE 2

Cloning of cDNA Encoding Rat NRSF

A. Construction of a cDNA Library

RNA was isolated from Rat1-EJ cells by standard procedures (Maniatis, T. et al., *Molecular Cloning: A Laboratory Manual,* Cold Spring Harbor Laboratory, Cold Spring Harbor, NY, 1982) and poly(A)$^+$ mRNA was selected using an "mRNA Separator" kit (Clontech Laboratories, Inc., Palo Alto, CA). cDNA was synthesized with the "Superscript" kit (GIBCO BRL Life Technologies, Inc., Gaithersburg, MD). Column-fractionated double-strand cDNA was ligated into a SalI-and NotI-digested pJT-2 plasmid vector, to yield plasmids containing cDNA inserts such as depicted in Figure 2. The pJT-2 plasmid vector was derived from the V19.8 vector (ATCC 68124). In particular, the HindIII and SacII cloning sites of V19.8 were changed into SalI and NotI sites by using synthetic oligonucleotide linkers to yield the PJT-2 vector. Plasmids containing cDNA inserts were transformed into DH10B *E. coli* cells by electroporation (Dower, W.J. et al., *Nucleic Acids Res. 16*:6127-6145, 1988).

B. Preparation of cDNA Probes and Isolation of Clones

Approximately 5 x $10^5$ primary transformants were screened with two oligonucleotide probes that were based on the combined amino acid sequences of the N-terminal region of NRSF as described in Example 1C, specifically, residues 5-24 (RGSRGKPGPAEGDPSPALPP) (SEQ ID NO:1), and residues 7-12 of the T40.4 tryptic peptide (GEYMCK) (SEQ ID NO:2). Their respective sequences (shown in antisense) were as follows ("N" indicates all four nucleotides):

```
(1) 5'-ATA GGG AAG GGC GGG GGA AGG GTC NCC CTC NGC AGG
                                   A       T
GCC GGG CTT GCC TCT GGA GCC TCT-3' (SEQ ID NO:16)

(2) 5'-TTT ACA CAT ATA TTC NCC-3' (SEQ ID NO:17)
       C   G       G   C
```

The synthetic oligonucleotides were end-labeled with $\alpha$-$^{32}$P-ATP with T4 polynucleotide kinase and used to screen replicate sets of nitrocellulose filters. The hybridization solution contained 6 x SSC, 50 mM sodium-

phosphate (pH 6.8), 0.1% sodium-pyrophosphate, 2 x Denhardt's solution, 50 μg/ml salmon sperm DNA and 20% formamide (for probe 1) or no formamide (for probe 2). Hybridization was carried out for fourteen hours at 42°C (for probe 1) or 37°C (for probe 2). The filters were washed at either 50°C with 0.5 x SSC/ 0.2% SDS/ 2 mM EDTA (for probe 1) or at 37°C with 2 x SSC/ 0.2% SDS/ 2 mM EDTA (for probe 2). Autoradiography of the filters gave ten clones that hybridized with both probes. These clones were purified by re-plating and probe hybridization as previously described.

EXAMPLE 3

Expression of Recombinant Rat NRSF by Transfected COS-7 Cells

In order to verify that the cDNA clones detected in the primary screening procedure described in Example 2B corresponded to NRSF-specific transcripts, the clones were transiently expressed in COS-7 monkey cells. For that purpose the cDNA clones were inserted into the pJT-2 eukaryotic expression vector under the control of the SV40 promoter and 3'-flanked with SV40 termination and poly-adenylation signals. The resulting plasmids, including the pJT-2/NRSF plasmid containing clone 44 cDNA (Fig. 2), were used to transfect COS-7 cells by electroporation as follows. 6 x $10^6$ cells in 0.8 ml Dulbecco's modified Eagle medium (DMEM) and 10% fetal bovine serum were transferred to a 0.4 cm cuvette and mixed with 20 μg of plasmid DNA in 10 μL of TE solution (10 mM Tris-HCl, pH 8.0, 1 mM EDTA). Electroporation was performed at room temperature, 1600 volts and 25 μF, using a BioRad Gene Pulser apparatus with the pulse controller unit set at 200 ohms. The cells were then diluted into 20 ml of DMEM/10% fetal bovine serum and transferred into a T75 flask (Falcon). After fourteen hours of incubation at 37°C, the medium was replaced with DMEM/1% fetal bovine serum, and the incubation was continued for an additional forty-eight hours.

The resulting conditioned media were then evaluated for their ability to stimulate tyrosine phosphorylation of the *neu* receptor in MDA-MB-453 human breast tumor cells as follows. The conditioned media were filtered through a 0.2-micron sterile filter unit (Costar, Cambridge, MA) and concentrated sixteen-fold by using a Centriprep 10 unit (Amicon, Beverly, MA). The concentrated media were diluted in phosphate-buffered saline (PBS) that contained 0.1% bovine serum albumin and were then added to individual wells of a forty eight-well dish that contained 3 x $10^5$ MDA-MB-453 cells per well. Following five minutes of incubation at 37°C, the media were aspirated and the cells were processed for Western blotting using a monoclonal antibody to phosphotyrosine (PY20). The protocol used for cell lysis and Western blotting is described in Peles, E. et al., *Cell* 69:205-216(1992).

The results of the analysis are depicted in Figure 3. Although the medium of untransfected COS-7 cells induced a slight increase in tyrosine phosphorylation, the medium harvested from clone 44 (i.e., pJT-2/NRSF plasmid) transfected cells was significantly more active. Therefore, clone 44 cDNA was completely purified by re-plating and by filter hybridization screening. Figure 3 (right panel) compares the activity of the completely purified clone 44 with the activity of control clones 27 and 29 that were randomly selected. Evidently, after transfection into COS-7 cells the completely purified clone 44 cDNA elicited higher activity than the control plasmids or the partially purified clones, including partially purified clone 44. On the basis of its hybridization to two independent oligonucleotide probes (Example 2B) and its ability to direct the synthesis of a biologically active NRSF, clone 44 was selected for further analysis by DNA sequencing.

EXAMPLE 4

Sequencing of Rat NRSF cDNA

Clone 44 cDNA was primarily sequenced using a 373A automated DNA sequencer and "Taq DyeDeoxy™ Terminator" cycle sequencing kits from Applied Biosystems, Inc. (Foster City, CA), in accordance with the manufacturer's instructions. Some of the sequencing was performed using $^{35}$S-dATP (Amersham) and "Sequenase™" kits from United States Biochemicals (Cleveland, Ohio), following manufacturer's instructions. Both strands of the cDNA of clone 44 cDNA were sequenced using synthetic oligonucleotides as primers. Nucleotide sequence analysis of the cDNA insert of clone 44 yielded a 1894 bp sequence that contained a 436 amino acid long open reading frame that extends to the 5' end of the cDNA. Downstream of the 3' end of this reading frame is a 594 base-long untranslated stretch. The latter includes a poly(A) tail preceded by a polyadenylation signal (AAATAAA). Because no stop codon and recognizable signal peptide were found at the amino terminus of the longest open reading frame, the nucleotide sequences of three other independent positive cDNA clones were analyzed in the same manner. All three clones added to the 5' end a 292 bp sequence that included an in-frame stop codon, suggesting that clone 44 is a 5' truncated cDNA that lacks 0.3 kilobases.

The combined nucleotide sequence of the cDNA clones is presented in Figure 4 and the clone 44 cDNA sequence is given in Figure 5. The combined sequence spans 2,186 base pairs, including a poly(A) tail, and contains an open reading frame of four hundred and twenty-two residues if the amino terminal methionine is considered to be the initiator codon. Hydropathy analysis (Fig. 6) revealed no prominent hydrophobic sequence at the amino terminus of the protein that could function as a signal peptide for protein secretion (von Hijne, G., *J. Mol . Biol. 184*:99-105, 1985). However, this analysis showed the presence of a highly hydrophobic stretch of twenty-three amino acids (underlined in Fig. 4) that qualifies as a potential transmembrane domain. This region bisects the presumed precursor of NRSF and defines a putative cytoplasmic domain of one hundred and fifty-seven amino acids. The predicted extracellular domain contains all of the peptide sequences that were determined directly from the purified protein (overlined in Fig. 4). They all perfectly matched the sequence deduced from the cDNA except for threonine residue 137, which was assigned as isoleucine in peptide T27.3 (Fig. 1). Five different cDNA clones encoded a threonine residue in the corresponding position, indicating that this change may not be due to an isoform of the protein. Instead, reexamination of the protein sequence data suggested that the isoleucine signal was carried over from the previous Edman cycle, and that the threonine escaped detection due to O-glycosylation. Significantly, all of these peptides sequenced are located in the amino-terminal half of the presumed ectodomain. The other half contains six cysteine residues that comprise an epidermal growth factor (EGF)- like domain (Fig. 7), which is known to be resistant to proteolysis due to its very compact structure (reviewed in Massagué, J., *J. Biol. Chem. 265:*21393-21396, 1990). The other two cysteine residues of the ectodomain, that appear to be disulfide-linked in NRSF (described in Example 1D), define an immunoglobulin (Ig) homology unit (Fig. 8). In addition, the predicted protein sequence includes four potential sites for N-linked glycosylation, all of which reside amino-terminally to the transmembrane region (indicated by asterisks in Fig. 4). A predicted overall structure for the NRSF precursor is presented in Figure 9.

By virtue of the transmembrane domain depicted in Figure 9, NRSF precursor is expected to accumulate on the surface of cells expressing high levels of NRSF mRNA. Membrane-bound precursor proteins have been found for other members of the EGF growth factor family. For example, Brachmann, R., et al. in *Cell 56*: 691-700, 1989, demonstrated the presence of transforming growth factor $\alpha$ on the surface of cells expressing transforming growth factor $\alpha$ mRNA. Molecules that specifically bind NRSF could selectively direct therapeutic molecules to cells overexpressing NRSF. For example, monoclonal antibodies raised against recombinant NRSF and conjugated to therapeutic molecules could selectively localize the therapeutic molecule on the surface of cells expressing high levels of membrane-bound NRSF. Such cells would include tumor cells with an activated *ras* gene. Antibody conjugates could be constructed using chemotherapeutic compounds, cytokines, toxins, lymphokines or radionuclides.

EXAMPLE 5

Functional Analyses of Recombinant NRSF Expressed by Transfected COS-7 Cells

It has been previously reported that homogeneously purified, naturally-occurring NRSF secreted from ras-transformed rat fibroblasts inhibits the growth of cultured human breast carcinoma cells and induces them to produce milk components indicative of cell differentiation (Peles, E. et al., *Cell 69*:205-216, 1992). In order to directly correlate these activities with the cloned DNA, the effects of a recombinant NRSF derived from clone 44 on cultured breast carcinoma cells were examined. More specifically, cultures of AU-565 or MDA-MB-453 cells were treated with sixteen-fold concentrated conditioned medium from COS-7 cells that had been transfected with either the clone 44 pJT-2/NRSF expression vector (Fig. 2) or a control pJT-2 plasmid that contained an unrelated cDNA insert (clone 27). Both media were used at 1:50 final dilution and incubated with the cells at 37°C for three days. Cell numbers were then determined with a hemocytometer and the nuclear area was measured by computerized image analysis. Staining for lipids and casein was performed as described in Bacus, S., et al., *Mol. Carcinogenesis 3*:350-362 (1990) . The experiment was repeated three times and yielded qualitatively similar results, which are set forth in Table 1.

TABLE 1

| | Cells with Lipid Droplets | Cell Number ($10^4/cm^2$) | Nuclear Area ($\mu^2$) | Cells with Casein (%) |
|---|---|---|---|---|
| AU-565 Cells | | | | |
| Control | 10% | $4 \times 10^4$ | 106 | <2 |
| Recombinant NRSF | 74% | $1.3 \times 10^4$ | 215.8 | >90 |
| MDA-MB-453 Cells | | | | |
| Control | 9% | $3.6 \times 10^4$ | 79.8 | >1 |
| Recombinant NRSF | 56% | $1.8 \times 10^4$ | 115.7 | 78 |

> means greater than
< means less than

The results show that AU-565 and MDA-MB-453 cultures that were treated with the control-conditioned medium displayed mostly an immature morphology, whereas most of the cells treated with conditioned medium containing the recombinant NRSF displayed a characteristic mature morphology that included large nuclei and the appearance of lipid droplets in the cytoplasm. Immunohistochemical staining specific for human casein (types β and χ) indicated that most of these cells also synthesized casein, unlike the control-treated cultures.

In conclusion, despite the fact that clone 44 lacks part of the 5' end of the NRSF cDNA, this cDNA clone directs synthesis of a functionally active NRSF which is produced by the transfected COS-7 cells.

This conclusion was further supported by the ability of recombinant NRSF to compete with naturally-occurring NRSF in ligand displacement analyses. Purified naturally-occurring rat NRSF was radiolabeled with 1 mCi of $Na^{125}I$ using Iodogen (Pierce, Rockford, IL) according to the manufacturer's instructions. Unreacted iodine was separated from the protein by gel filtration on Excellulose GF-5 desalting column (Pierce). The specific activity of the radiolabeled naturally-occurring NRSF ($^{125}I$-NRSF) was 3 x $10^6$ cpm/ng. The radiolabeled NRSF (10 picomolar) was incubated for sixty minutes at 4°C with monolayers of MDA-MB-453 cells that were grown in a 24-well dish (Costar). This incubation was also performed in the presence of conditioned media from transfected COS-7 cells. Unbound $^{125}I$-NRSF was removed by three washes with phosphate buffered saline (PBS), and the cells were solubilized in 0.1 N NaOH solution containing 0.1% SDS. Radioactivity was determined with a $\gamma$-counter.

As depicted in Figure 10, conditioned medium containing recombinant NRSF expressed from the pJT-2/NRSF expression vector (Fig. 2) in COS-7 cells reduced the total $^{125}I$-NRSF binding by approximately 50%. For a negative control, COS-7 conditioned medium from cells transfected with a pJT-2 expression vector containing a rat TGF$\alpha$ cDNA (Blasband, A. et al., *Mol. Cell Biol. 10:* 2111-2121, 1990) was employed. In contrast to the recombinant NRSF conditioned medium, the TGF$\alpha$ conditioned medium did not inhibit $^{125}I$-NRSF binding. The partial inhibitory effect of the recombinant NRSF conditioned medium, as compared with the purified, natural, unlabeled NRSF (Peles, E. et al., *Cell 69*:205-216, 1992), may be attributed to its relatively low concentration in the binding assay. In addition, it could be due to high non-specific ligand binding.

To overcome this problem and also demonstrate direct interaction with *neu* receptor, a covalent crosslinking assay was employed. This was performed by allowing MDA-MB-453 cells to bind $^{125}I$-NRSF in the presence of transfected COS-7 cell conditioned media, as above. The chemical crosslinking reagent bis (sulfosuccinimidyl) suberate ($BS^3$, Pierce) was added at 1 mM final concentration after one hour of binding at 4°C, followed by a brief wash with PBS. Following forty-five minutes of incubation at 22°C, the monolayers were incubated for ten minutes with quenching buffer (100 mM glycine in PBS, pH 7.4). The cells were then washed twice with ice-cold PBS, lysed in lysis buffer, and the *neu* protein was immunoprecipitated with monoclonal antibody Ab-3 according to the protocol described in Peles, E. et al., *EMBO J. 10*:2077-2086 (1991). The extensively washed immunocomplexes were resolved by gel electrophoresis (6% acrylamide) and autoradiography. The results of this analysis (Fig. 11) indicated that the recombinant NRSF, unlike recombinant TGF$\alpha$, was able to displace most of the receptor-bound $^{125}I$-NRSF. This result confirmed that the clone 44 cDNA encodes a functional NRSF molecule.

EXAMPLE 6

Northern Blot Analyses of NRSF Expression

To determine the size and tissue distribution of the NRSF mRNA, Northern blot hybridization experiments were carried out using the cDNA insert of clone 44 as a hybridization probe. Tissues were obtained through surgery of adult female rats, their RNA was extracted and poly(A) + RNA was selected using standard methods (Manniatis, T. et al., *Molecular Cloning*: A Laboratory Manual, Cold Spring Harbor Laboratory, Cold Spring Harbor, New York, 1982). The 1.9 kb-long cDNA insert of clone 44 was labeled with $\alpha$-$^{32}$P-dCTP by the random priming method (Feinberg, A.P., and Vogelstein, B., *Anal. Biochem 132*:6-13, 1983). The conditions of hybridization were as follows: 6 x SSC, 50 mM sodium phosphate (pH 6.8), 0.1% sodium pyrophosphate, 2 x Denhardt's solution, 50 $\mu$g/ml salmon sperm DNA and 50% formamide. Hybridization was carried out for fourteen hours at 42°C, followed by washing for thirty minutes at 60°C with 0.2 x SSC, 0.1% SDS and 2 mM EDTA. The filters were exposed to Kodak XAR x-ray film with an intensifier screen at -70°C for the indicated periods of time.

Figure 12 (panel A) shows that three bands were visualized in the Northern blots with poly(A) selected RNA of Rat1-EJ fibroblasts. Their molecular sizes corresponded to 6.8, 2.6 and 1.7 kilobases. After further autoradiography, two additional species of mRNA became visible (not shown). The relative level of expression of NRSF in normal Rat1 or 3T3 fibroblasts was significantly lower than in the ras-transformed cells, in agreement with the earlier observation that correlated the *neu* receptor stimulatory activity with transformation by an oncogenic *ras* gene (Yarden, Y., and Weinberg, R., *Proc. Natl. Acad. Sci. USA 86:* 3179-3188, 1989).

In a survey of adult rat tissues (Figure 12, panel B), the highest NRSF mRNA expression was observed in the spinal cord. NRSF mRNAs were also detected in brain tissue. In addition to the mRNAs described above, these tissues express a variant mRNA that is 3.4 kb in size. The *neu* receptor, which is stimulated by NRSF, is present in human fetal spinal cord and brain (Quirke, P. et al., *Br. J. Cancer 60*: 64-69, 1989), which suggests that expression of both NRSF and *neu* receptor modulates the growth and differentiation of neural tissue. Oncogenic activation of the *neu* receptor gene in rat neuroblastomas (Bargmann, C. et al., *Nature 319:* 226-230, 1986), further implicates the *neu* receptor and NRSF in neural tissue growth regulation.

NRSF expression, by virtue of its *neu* receptor stimulatory activity, may also regulate the growth and differentiation of other cell types. The *neu* receptor is found on the surface of normal differentiated human epithelial cells of the gastrointestinal, respiratory, urinary and reproductive tracts, as well as skin, and is also expressed in corresponding human fetal tissues (Press, M. et al., *Oncogene* 5: 953-962, 1990) . Figure 12, panel B demonstrates tissue-specific regulation of NRSF mRNA expression levels in adult rat tissues. Positive tissues include lung, ovary and stomach. Relatively low amounts of the middle-size transcript were displayed by the skin, kidney and heart. The liver, spleen and muscle did not contain detectable NRSF mRNA. Figure 12, panel B also shows tissue-specific variation in the relative proportion of the different NRSF mRNA species. Each variant mRNA could encode variant NRSF proteins with different biological properties. Natural NRSF may regulate cell proliferation and differentiation through tissue-specific variations in NRSF mRNA structure and expression levels. Administration of the non-natural NRSF of this invention, particularly to injured or diseased tissues lacking normal levels of natural NRSF protein, can provide *neu* receptor stimulation and may modulate cell growth and differentiation in the treated tissue. In particular, heart, stomach, brain, spinal cord, ovary, lung, kidney and skin tissues express naturally-occurring NRSF and would be expected to respond to treatment with the recombinant NRSF of this invention.

Abnormal NRSF or *neu* receptor expression may lead to autocrine interactions that contribute to neoplasia (Browder, T. et al., *Cancer Cells 1:* 9-17, 1989; Yarden, Y., and Ullrich, A., *Ann. Rev. Biochem. 57:* 443-448, 1988). Overexpression or amplification of the *neu* receptor gene occurs in human tumors derived from ovary, lung and stomach tissues (Slamon, D. et al., *Science 244:* 707-712, 1989; Tal, M. et al., *Cancer Res. 48:* 1517-1520, 1988; Cline, M. et al., *Cancer 60:* 2669 et *seq.,* 1987). These tissues express neu receptor stimulating factor (Figure 12, panel B), which may affect the growth of the tumors. Figure 12, panel C, shows that human tumor cells can overexpress NRSF mRNA. An initial survey of human tumor cell lines found that HT-1080 fibrosarcoma cells, Hs 294T melanoma cells and MDA-MB-231 mammary adenocarcinoma cells express elevated NRSF mRNA levels (Figure 12, panel C). Perturbations in the expression of either the *neu* receptor or its cognate *neu* receptor stimulatory factor can apparently alter normal cell growth and differentiation, with pathological consequences such as tumor development. The recombinant NRSF of this invention may be used to restore a balanced interaction between *neu* receptor and NRSF in cases where natural NRSF is deficient, or when the *neu* receptor is overexpressed.

Abnormal tumor cell expression of NRSF may result from activated *ras* oncogenes. NRSF mRNA expression increases dramatically in cells transformed with a *ras* oncogene (Figure 12, panel A). Activated *ras* genes are found in many carcinoma cell lines and in human tumors from several organs, including colon, lung, gall bladder, urinary bladder, and pancreas carcinomas, as well as melanomas, sarcomas and leukemias (Pimmental, E., *Oncogenes*, 2nd Edition, Volume II, CRC Press, Boca Raton, FL, 1989). Activated *ras* mutations are also found in premalignant tissues, such as human colorectal adenomas, and are thought to contribute to tumorigenesis (Fearon, E., and Vogelstein, B., *Cell 61:* 759-767, 1990). Elevated NRSF expression in malignant and premalignant human tissues, particularly those containing activated *ras* genes, can be found with the NRSF DNA and NRSF mRNA detection methods of this invention. Other methods can also identify elevated NRSF expression in human tissues. These methods, known to those skilled in the art of analyzing protein and mRNA expression, include immunohistochemical assays, radioimmunoassays, enzyme-linked immunoabsorbent assays, and polymerase chain reaction assays. The identification of premalignant and malignant cells overexpressing NRSF is particularly useful for the diagnosis of human cancer.

SEQUENCE LISTING

(1) GENERAL INFORMATION:

(i) APPLICANTS: Wen, D.
Yarden, Y.
Peles, E.

(ii) TITLE OF INVENTION: Recombinant Stimulating Factor of the neu Receptor

(iii) NUMBER OF SEQUENCES: 17

(iv) CORRESPONDENCE ADDRESS:

(A) ADDRESSEE: Amgen Inc.

(B) STREET: Amgen Center
1840 Dehavilland Drive

(C) CITY: Thousand Oaks

(D) STATE: California

(E) COUNTRY: USA

(F) ZIP: 91320-1789

(v) COMPUTER READABLE FORM:

(A) MEDIUM TYPE: Diskette, 3.5 in., DS, 1.4 MB

(B) COMPUTER: Apple Macintosh

(C) OPERATING SYSTEM: Macintosh OS 7.0.

(D) SOFTWARE: Microsoft Word Version 5.1

(vi) CURRENT APPLICATION DATA:

(A) APPLICATION NUMBER: 07/877431

(B) FILING DATE: 29-04-1992

(C) CLASSIFICATION:

(2) INFORMATION FOR SEQ ID NO:1:

(i) SEQUENCE CHARACTERISTICS:

(A) LENGTH: 20 amino acid residuess

(B) TYPE: amino acid

(xi) SEQUENCE DESCRIPTION: SEQ ID NO:1:

Arg Gly Ser Arg Gly Lys Pro Gly Pro Ala Glu Gly Asp Pro Ser Pro 16

Ala Leu Pro Pro 20

(3) INFORMATION FOR SEQ ID NO:2:

(i) SEQUENCE CHARACTERISTICS:

(A) LENGTH: 6 amino acid residues

(B) TYPE: amino acid

(xi) SEQUENCE DESCRIPTION: SEQ ID NO:2:

Gly Glu Tyr Met Cys Lys 6

(4) INFORMATION FOR SEQ ID NO:3:

(i) SEQUENCE CHARACTERISTICS:

(A) LENGTH: 2186 base pairs

(B) TYPE: nucleic acid

(C) STRANDEDNESS: double stranded

(D) TOPOLOGY: unknown

(xi) SEQUENCE DESCRIPTION: SEQ ID NO:3:

```
AGCTGCCGGG AGATGCGAGC GCAGACCGGA TTGTGATCAC CTTTCCCTCT TCGGGCTGTA    60

AGAGAGCGAG ACAAGCCACC GAAGCGAGGC CACTCCAGAG CCGGCAGCGG AGGGACCCGG   120

GACACTAGAG CAGCTCCGAG CCACTCCAGA CTGAGCGGAC GCTCCAGGTG ATCGAGTCCA   180

CGCTGCTTCC TGCAGGCGAC AGGCGACGCC TCCCGAGCAG CCCGGCCACT GGCTCTTCCC   240

CTCCTGGGAC AAACTTTTCT GCAAGCCCTT GGACCAAACT TGTCGCGCGT CACCGTCACC   300

CAACCGGGTC CGCGTAGAGC GCTCATCTTC GGCGAG ATG TCT GAG CGC AAA GAA      354
                                        Met Ser Glu Arg Lys Glu

GGC AGA GGC AAG GGG AAG GGC AAG AAG AAG GAC CGG GGA TCC CGC GGG      402
Gly Arg Gly Lys Gly Lys Gly Lys Lys Lys Asp Arg Gly Ser Arg Gly

AAG CCC GGG CCC GCC GAG GGC GAC CCG AGC CCA GCA CTG CCT CCC AGA      450
Lys Pro Gly Pro Ala Glu Gly Asp Pro Ser Pro Ala Leu Pro Pro Arg

TTG AAA GAA ATG AAG AGC CAG GAG TCA GCT GCA GGC TCC AAG CTA GTG      498
Leu Lys Glu Met Lys Ser Gln Glu Ser Ala Ala Gly Ser Lys Leu Val
```

```
CTC CGG TGC GAA ACC AGC TCC GAG TAC TCC TCA CTC AGA TTC AAA TGG      546
Leu Arg Cys Glu Thr Ser Ser Glu Tyr Ser Ser Leu Arg Phe Lys Trp

TTC AAG AAT GGG AAC GAG CTG AAC CGC AAA AAT AAA CCA GAA AAC ATC      594
Phe Lys Asn Gly Asn Glu Leu Asn Arg Lys Asn Lys Pro Glu Asn Ile

AAG ATA CAG AAG AAG CCA GGG AAG TCA GAG CTT CGA ATT AAC AAA GCA      642
Lys Ile Gln Lys Lys Pro Gly Lys Ser Glu Leu Arg Ile Asn Lys Ala

TCC CTG GCT GAC TCT GGA GAG TAT ATG TGC AAA GTG ATC AGC AAG TTA      690
Ser Leu Ala Asp Ser Gly Glu Tyr Met Cys Lys Val Ile Ser Lys Leu

GGA AAT GAC AGT GCC TCT GCC AAC ATC ACC ATT GTT GAG TCA AAC GAG      738
Gly Asn Asp Ser Ala Ser Ala Asn Ile Thr Ile Val Glu Ser Asn Glu

TTC ATC ACT GGC ATG CCA GCC TCG ACT GAG ACA GCC TAT GTG TCC TCA      786
Phe Ile Thr Gly Met Pro Ala Ser Thr Glu Thr Ala Tyr Val Ser Ser

GAG TCT CCC ATT AGA ATC TCA GTT TCA ACA GAA GGC GCA AAC ACT TCT      834
Glu Ser Pro Ile Arg Ile Ser Val Ser Thr Glu Gly Ala Asn Thr Ser

TCA TCC ACA TCA ACA TCC ACG ACT GGG ACC AGC CAT CTC ATA AAG TGT      882
Ser Ser Thr Ser Thr Ser Thr Thr Gly Thr Ser His Leu Ile Lys Cys

GCG GAG AAG GAG AAA ACT TTC TGT GTG AAT GGG GGC GAG TGC TTC ACG      930
Ala Glu Lys Glu Lys Thr Phe Cys Val Asn Gly Gly Glu Cys Phe Thr

GTG AAG GAC CTG TCA AAC CCG TCA AGA TAC TTG TGC AAG TGC CAA CCT      978
Val Lys Asp Leu Ser Asn Pro Ser Arg Tyr Leu Cys Lys Cys Gln Pro

GGA TTC ACT GGA GCA AGA TGT ACT GAG AAT GTA CCC ATG AAA GTC CAA      1026
Gly Phe Thr Gly Ala Arg Cys Thr Glu Asn Val Pro Met Lys Val Gln

ACC CAA GAA AAA GCG GAG GAA CTC TAC CAG AAG AGG GTG CTG ACA ATT      1074
Thr Gln Glu Lys Ala Glu Glu Leu Tyr Gln Lys Arg Val Leu Thr Ile

ACT GGC ATC TGT ATC GCC CTG CTG GTG GTC GGC ATC ATG TGT GTG GTG      1122
Thr Gly Ile Cys Ile Ala Leu Leu Val Val Gly Ile Met Cys Val Val

GCC TAC TGC AAA ACC AAG AAG CAG CGG CAG AAG CTT CAT GAT CGG CTT      1170
Ala Tyr Cys Lys Thr Lys Lys Gln Arg Gln Lys Leu His Asp Arg Leu

CGG CAG AGT CTT CGG TCA GAA CGG AGC AAC CTG GTG AAC ATA GCG AAT      1210
Arg Gln Ser Leu Arg Ser Glu Arg Ser Asn Leu Val Asn Ile Ala Asn
```

```
GGG CCT CAC CAC CCA AAC CCA CCG CCA GAG AAC GTG CAG CTG GTG AAT      1266
Gly Pro His His Pro Asn Pro Pro Pro Glu Asn Val Gln Leu Val Asn

CAA TAC GTA TCT AAA AAC GTC ATC TCC AGT GAG CAT ATT GTT GAG AGA      1314
Gln Tyr Val Ser Lys Asn Val Ile Ser Ser Glu His Ile Val Glu Arg

GAA GTG GAG ACT TCC TTT TCC ACC AGT CAT TAC ACT TCC ACA GCC CAT      1362
Glu Val Glu Thr Ser Phe Ser Thr Ser His Tyr Thr Ser Thr Ala His

CAC TCC ACG ACT GTC ACC CAG ACT CCT AGT CAC AGC TGG AGT AAT GGG      1410
His Ser Thr Thr Val Thr Gln Thr Pro Ser His Ser Trp Ser Asn Gly

CAC ACG GAG AGC GTC ATT TCA GAA AGC AAC TCC GTA ATC ATG ATG TCT      1458
His Thr Glu Ser Val Ile Ser Glu Ser Asn Ser Val Ile Met Met Ser

TCG GTA GAG AAC AGC AGG CAC AGC AGT CCC GCC GGG GGC CCA CGA GGA      1506
Ser Val Glu Asn Ser Arg His Ser Ser Pro Ala Gly Gly Pro Arg Gly

CGT CTT CAT GGC CTG GGA GGC CCT CGT GAT AAC AGC TTC CTC AGG CAT      1554
Arg Leu His Gly Leu Gly Gly Pro Arg Asp Asn Ser Phe Leu Arg His

GCC AGA GAA ACC CCT GAC TCC TAC AGA GAC TCT CCT CAT AGC GAA AGG      1602
Ala Arg Glu Thr Pro Asp Ser Tyr Arg Asp Ser Pro His Ser Glu Arg

TAAAATGGAA GGGTAAAGCT ATCGTGGAGG AGAACCTCAT TCAGTGAGAG AATCCCATGA    1662

GCACCTGCGG TCTCTCCTAA GGAAACTCAT CCTTTCATAA GGGGCGTCAT CAATTCCCTG    1722

ACGCTCCTAG TTGATGAAGT CAACCTCTTA TTTGTCTGAA CTCCTTTCTC CTGAGCTTCT    1782

CCCCGCGTCC CAGTGGCTGA CAGGCAACCA ACTCCTAAAG AGCAGGAGTA ATGTAATGTG    1842

GAAGGCCCAG CCGACTTGGA GTTCTCAGAC CTGACCCTAA GGTCAGACTC ACTGGGGTCT    1902

TCCTTTCGTC AGGTGCACCA TTTTAAGGAC CCTCATCTAA TCCCGATCAG CCAGTTGTCC    1962

ATTCTCTACC TCGATGGTTG TTCTGCCCTC CTCTCCTCAT GCTCTAAGTA CCCAGCCTCT    2022

AGCCTCAGTT AAATCAAGTC GAGGGCTGGG ACACGGCTGA TGGTCATGGC GGAATGCCTC    2082

CTCGGGTTCC ACCACACTGT ATCCATTTAC GACATCCCTA GAAGTCATGG GTAAATAAAG    2142

TTGTTGTGTG ATGTGAAAAA AAAAAAAAAA AAAAAAAAAA AAAA                     2186
```

(5) INFORMATION FOR SEQ ID NO:4:

(i) SEQUENCE CHARACTERISTICS:

(A) LENGTH: 1894 base pairs

(B) TYPE: nucleic acid

(C) STRANDEDNESS: double stranded

(D) TOPOLOGY: unknown

(xi) SEQUENCE DESCRIPTION: SEQ ID NO:4:

```
CCGT CACCCAACCG GGTCCGCGTA GAGCGCTCAT CTTCGGCGAG ATG TCT GAG      53
                                                 Met Ser Glu

CGC AAA GAA GGC AGA GGC AAG GGG AAG GGC AAG AAG AAG GAC CGG GGA    101
Arg Lys Glu Gly Arg Gly Lys Gly Lys Gly Lys Lys Lys Asp Arg Gly

TCC CGC GGG AAG CCC GGG CCC GCC GAG GGC GAC CCG AGC CCA GCA CTG    149
Ser Arg Gly Lys Pro Gly Pro Ala Glu Gly Asp Pro Ser Pro Ala Leu

CCT CCC AGA TTG AAA GAA ATG AAG AGC CAG GAG TCA GCT GCA GGC TCC    197
Pro Pro Arg Leu Lys Glu Met Lys Ser Gln Glu Ser Ala Ala Gly Ser

AAG CTA GTG CTC CGG TGC GAA ACC AGC TCC GAG TAC TCC TCA CTC AGA    245
Lys Leu Val Leu Arg Cys Glu Thr Ser Ser Glu Tyr Ser Ser Leu Arg

TTC AAA TGG TTC AAG AAT GGG AAC GAG CTG AAC CGC AAA AAT AAA CCA    293
Phe Lys Trp Phe Lys Asn Gly Asn Glu Leu Asn Arg Lys Asn Lys Pro

GAA AAC ATC AAG ATA CAG AAG AAG CCA GGG AAG TCA GAG CTT CGA ATT    341
Glu Asn Ile Lys Ile Gln Lys Lys Pro Gly Lys Ser Glu Leu Arg Ile

AAC AAA GCA TCC CTG GCT GAC TCT GGA GAG TAT ATG TGC AAA GTG ATC    389
Asn Lys Ala Ser Leu Ala Asp Ser Gly Glu Tyr Met Cys Lys Val Ile

AGC AAG TTA GGA AAT GAC AGT GCC TCT GCC AAC ATC ACC ATT GTT GAG    437
Ser Lys Leu Gly Asn Asp Ser Ala Ser Ala Asn Ile Thr Ile Val Glu

TCA AAC GAG TTC ATC ACT GGC ATG CCA GCC TCG ACT GAG ACA GCC TAT    485
Ser Asn Glu Phe Ile Thr Gly Met Pro Ala Ser Thr Glu Thr Ala Tyr

GTG TCC TCA GAG TCT CCC ATT AGA ATC TCA GTT TCA ACA GAA GGC GCA    533
Val Ser Ser Glu Ser Pro Ile Arg Ile Ser Val Ser Thr Glu Gly Ala
```

AAC ACT TCT TCA TCC ACA TCA ACA TCC ACG ACT GGG ACC AGC CAT CTC 581
Asn Thr Ser Ser Ser Thr Ser Thr Ser Thr Thr Gly Thr Ser His Leu

ATA AAG TGT GCG GAG AAG GAG AAA ACT TTC TGT GTG AAT GGG GGC GAG 629
Ile Lys Cys Ala Glu Lys Glu Lys Thr Phe Cys Val Asn Gly Gly Glu

TGC TTC ACG GTG AAG GAC CTG TCA AAC CCG TCA AGA TAC TTG TGC AAG 677
Cys Phe Thr Val Lys Asp Leu Ser Asn Pro Ser Arg Tyr Leu Cys Lys

TGC CAA CCT GGA TTC ACT GGA GCA AGA TGT ACT GAG AAT GTA CCC ATG 725
Cys Gln Pro Gly Phe Thr Gly Ala Arg Cys Thr Glu Asn Val Pro Met

AAA GTC CAA ACC CAA GAA AAA GCG GAG GAA CTC TAC CAG AAG AGG GTG 773
Lys Val Gln Thr Gln Glu Lys Ala Glu Glu Leu Tyr Gln Lys Arg Val

CTG ACA ATT ACT GGC ATC TGT ATC GCC CTG CTG GTG GTC GGC ATC ATG 821
Leu Thr Ile Thr Gly Ile Cys Ile Ala Leu Leu Val Val Gly Ile Met

TGT GTG GTG GCC TAC TGC AAA ACC AAG AAG CAG CGG CAG AAG CTT CAT 869
Cys Val Val Ala Tyr Cys Lys Thr Lys Lys Gln Arg Gln Lys Leu His

GAT CGG CTT CGG CAG AGT CTT CGG TCA GAA CGG AGC AAC CTG GTG AAC 917
Asp Arg Leu Arg Gln Ser Leu Arg Ser Glu Arg Ser Asn Leu Val Asn

ATA GCG AAT GGG CCT CAC CAC CCA AAC CCA CCG CCA GAG AAC GTG CAG 965
Ile Ala Asn Gly Pro His His Pro Asn Pro Pro Pro Glu Asn Val Gln

CTG GTG AAT CAA TAC GTA TCT AAA AAC GTC ATC TCC AGT GAG CAT ATT 1013
Leu Val Asn Gln Tyr Val Ser Lys Asn Val Ile Ser Ser Glu His Ile

GTT GAG AGA GAA GTG GAG ACT TCC TTT TCC ACC AGT CAT TAC ACT TCC 1061
Val Glu Arg Glu Val Glu Thr Ser Phe Ser Thr Ser His Tyr Thr Ser

ACA GCC CAT CAC TCC ACG ACT GTC ACC CAG ACT CCT AGT CAC AGC TGG 1109
Thr Ala His His Ser Thr Thr Val Thr Gln Thr Pro Ser His Ser Trp

AGT AAT GGG CAC ACG GAG AGC GTC ATT TCA GAA AGC AAC TCC GTA ATC 1157
Ser Asn Gly His Thr Glu Ser Val Ile Ser Glu Ser Asn Ser Val Ile

ATG ATG TCT TCG GTA GAG AAC AGC AGG CAC AGC AGT CCC GCC GGG GGC 1205
Met Met Ser Ser Val Glu Asn Ser Arg His Ser Ser Pro Ala Gly Gly

CCA CGA GGA CGT CTT CAT GGC CTG GGA GGC CCT CGT GAT AAC AGC TTC 1253
Pro Arg Gly Arg Leu His Gly Leu Gly Gly Pro Arg Asp Asn Ser Phe

```
CTC AGG CAT GCC AGA GAA ACC CCT GAC TCC TAC AGA GAC TCT CCT CAT   1301
Leu Arg His Ala Arg Glu Thr Pro Asp Ser Tyr Arg Asp Ser Pro His

AGC GAA AGG TAAAATGGAA GGGTAAAGCT ATCGTGGAGG AGAACCTCAT TCAGTGAGAG  1360
Ser Glu Arg

AATCCCATGA GCACCTGCGG TCTCTCCTAA GGAAACTCAT CCTTTCATAA GGGGCGTCAT  1420
CAATTCCCTG ACGCTCCTAG TTGATGAAGT CAACCTCTTA TTTGTCTGAA CTCCTTTCTC  1480
CTGAGCTTCT CCCCGCGTCC CAGTGGCTGA CAGGCAACCA ACTCCTAAAG AGCAGGAGTA  1540
ATGTAATGTG GAAGGCCCAG CCGACTTGGA GTTCTCAGAC CTGACCCTAA GGTCAGACTC  1600
ACTGGGGTCT TCCTTTCGTC AGGTGCACCA TTTTAAGGAC CCTCATCTAA TCCCGATCAG  1660
CCAGTTGTCC ATTCTCTACC TCGATGGTTG TTCTGCCCTC CTCTCCTCAT GCTCTAAGTA  1720
CCCAGCCTCT AGCCTCAGTT AAATCAAGTC GAGGGCTGGG ACACGGCTGA TGGTCATGGC  1780
GGAATGCCTC CTCGGGTTCC ACCACACTGT ATCCATTTAC GACATCCCTA GAAGTCATGG  1840
GTAAATAAAG TTGTTGTGTG ATGTGAAAAA AAAAAAAAAA AAAAAAAAAA AAAA        1894
```

(6) INFORMATION FOR SEQ ID NO:5:

(i) SEQUENCE CHARACTERISTICS:

(A) LENGTH: 64 amino acid residues

(B) TYPE: amino acid

(xi) SEQUENCE DESCRIPTION: SEQ ID NO:5:

```
Cys Ala Glu Lys Glu Lys Thr Phe Cys Val Asn Gly Gly Glu Cys Phe   16
Thr Val Lys Asp Leu Ser Asn Pro Ser Arg Tyr Leu Cys Lys Cys Gln   32
Pro Gly Phe Thr Gly Ala Arg Cys Thr Glu Asn Val Pro Met Lys Val   48
Gln Thr Gln Glu Lys Ala Glu Glu Leu Tyr Gln Lys Arg Val Leu Thr   64
```

(7) INFORMATION FOR SEQ ID NO:6:

(i) SEQUENCE CHARACTERISTICS:

(A) LENGTH: 60 amino acid residues

(B) TYPE: amino acid

(xi) SEQUENCE DESCRIPTION: SEQ ID NO:6:

Cys Pro Asp Ser His Thr Gln Tyr Cys Phe His Gly Thr Cys Arg Phe 16

Leu Val Gln Glu Glu Lys Pro Ala Cys Val Cys His Ser Gly Tyr Val 32

Gly Val Arg Cys Glu His Ala Asp Leu Leu Ala Val Val Ala Ala Ser 48

Gln Lys Lys Gln Ala Ile Thr Ala Ala Val Val Val 60

(8) INFORMATION FOR SEQ ID NO:7:

(i) SEQUENCE CHARACTERISTICS:

(A) LENGTH: 60 amino acid residues

(B) TYPE: amino acid

(xi) SEQUENCE DESCRIPTION: SEQ ID NO:7:

Cys Asn Ala Glu Phe Gln Asn Phe Cys Ile His Gly Glu Cys Lys Tyr 16

Ile Glu His Leu Glu Ala Val Thr Cys Asn Cys Gln Gln Glu Tyr Phe 32

Gly Glu Arg Cys Gly Glu Lys Ser Met Lys Thr His Ser Met Ile Asp 48

Ser Ser Leu Ser Lys Ile Ala Leu Leu Ala Ile Ala 60

(9) INFORMATION FOR SEQ ID NO:8:

(i) SEQUENCE CHARACTERISTICS:

(A) LENGTH: 61 amino acid residues

(B) TYPE: amino acid

(xi) SEQUENCE DESCRIPTION: SEQ ID NO:8:

Cys Pro Ser Ser Tyr Asp Gly Tyr Cys Leu Asn Gly Gly Val Cys Met 16

His Ile Glu Ser Leu Asp Ser Tyr Thr Cys Asn Cys Val Ile Gly Tyr 32

Ser Gly Asp Arg Cys Gln Thr Arg Asp Leu Arg Trp Trp Glu Leu Arg 48

His Ala Gly Tyr Gly Gln Lys His Asp Ile Met Val Val 61

(10) INFORMATION FOR SEQ ID NO:9:

(i) SEQUENCE CHARACTERISTICS:

(A) LENGTH: 60 amino acid residues

(B) TYPE: amino acid

(xi) SEQUENCE DESCRIPTION: SEQ ID NO:9:

Cys Leu Arg Lys Tyr Lys Asp Phe Cys Ile His Gly Glu Cys Lys Tyr 16

Val Lys Glu Leu Arg Ala Pro Ser Cys Ile Cys His Pro Gly Tyr His 32

Gly Glu Arg Cys His Gly Leu Ser Leu Pro Val Glu Asn Arg Val Tyr 48

Thr Tyr Asp His Thr Thr Ile Leu Ala Val Val Ala 60

(11) INFORMATION FOR SEQ ID NO:10:

(i) SEQUENCE CHARACTERISTICS:

(A) LENGTH: 60 amino acid residues

(B) TYPE: amino acid

(xi) SEQUENCE DESCRIPTION: SEQ ID NO:10:

Cys Ala Ala Lys Phe Gln Asn Phe Cys Ile His Gly Glu Cys Arg Tyr 16

Ile Glu Asn Leu Glu Val Val Thr Cys His Cys His Gln Asp Tyr Phe 32

Gly Glu Arg Cys Gly Glu Lys Thr Met Lys Thr Gln Lys Lys Asp Asp 48

Ser Asp Leu Ser Lys Ile Ala Leu Ala Ala Ile Ile 60

(12) INFORMATION FOR SEQ ID NO:11:

(i) SEQUENCE CHARACTERISTICS:

(A) LENGTH: 60 amino acid residues

(B) TYPE: amino acid

(xi) SEQUENCE DESCRIPTION: SEQ ID NO:11:

Cys Gly Pro Glu Gly Asp Gly Tyr Cys Leu His Gly Asp Cys Ile His 16

Ala Arg Asp Ile Asp Gly Met Tyr Cys Arg Cys Ser His Gly Tyr Thr 32

Gly Ile Arg Cys Gln His Val Val Leu Val Asp Tyr Gln Arg Ser Glu 48

Asn Pro Asn Thr Thr Thr Ser Tyr Ile Pro Ser Pro 60

(13) INFORMATION FOR SEQ ID NO:12:

(i) SEQUENCE CHARACTERISTICS:

(A) LENGTH: 48 amino acid residues

(B) TYPE: amino acid

(xi) SEQUENCE DESCRIPTION: SEQ ID NO:12:

Cys Asn His Asp Tyr Glu Asn Tyr Cys Leu Asn Asn Gly Thr Cys Phe 16

Thr Ile Ala Leu Asp Asn Val Ser Ile Thr Pro Phe Cys Val Cys Arg 32

Ile Asn Tyr Glu Gly Ser Arg Cys Gln Phe Ile Asn Leu Val Thr Tyr 48

(14) INFORMATION FOR SEQ ID NO:13:

(i) SEQUENCE CHARACTERISTICS:

(A) LENGTH: 49 amino acid residues

(B) TYPE: amino acid

(xi) SEQUENCE DESCRIPTION: SEQ ID NO:13:

Cys Asn Asp Asp Tyr Lys Asn Tyr Cys Leu Asn Asn Gly Thr Cys Phe 16

Thr Val Ala Leu Asn Asn Val Ser Leu Asn Pro Phe Cys Ala Cys His 32

Ile Asn Tyr Val Gly Ser Arg Cys Gln Phe Ile Asn Leu Ile Thr Ile 48

Lys 49

(15) INFORMATION FOR SEQ ID NO:14:

(i) SEQUENCE CHARACTERISTICS:

(A) LENGTH: 65 amino acid residues

(B) TYPE: amino acid

(xi) SEQUENCE DESCRIPTION: SEQ ID NO:14:

Leu Val Leu Arg Cys Glu Thr Ser Ser Glu Tyr Ser Ser Leu Arg Phe 16

Arg Trp Phe Lys Asn Gly Asn Glu Leu Asn Arg Lys Asn Asn Lys Pro 32

Glu Asn Ile Lys Ile Gln Lys Lys Pro Gly Lys Ser Glu Leu Arg Ile 48

Asn Lys Ala Ser Leu Ala Asp Ser Gly Glu Tyr Met Cys Lys Val Ile 64

Ser 65

(16) INFORMATION FOR SEQ ID NO:15:

(i) SEQUENCE CHARACTERISTICS:

(A) LENGTH: 65 amino acid residues

(B) TYPE: amino acid

(xi) SEQUENCE DESCRIPTION: SEQ ID NO:15:

Val Thr Leu Thr Cys Glu Ala Ser Gly Asp Pro Ile Pro Ser Ile Thr 16

Trp Arg Thr Ser Thr Arg Asn Ile Ser Ser Glu Glu Gln Asp Leu Asp 32

Gly His Met Val Val Arg Ser His Ala Arg Val Ser Ser Leu Thr Leu 48

Lys Ser Ile Gln Tyr Arg Asp Ala Gly Glu Tyr Met Cys Thr Ala Ser 64

Asn 65

(17) INFORMATION FOR SEQ ID NO:16:

(i) SEQUENCE CHARACTERISTICS:

(A) LENGTH: 60 base pairs

(B) TYPE: nucleic acid

(C) STRANDEDNESS: single stranded

(D) TOPOLOGY: linear

(xi) SEQUENCE DESCRIPTION: SEQ ID NO:16:

ATA GGG AAG GGC GGG GGA AGG RTC NCC YTC NGC AGG GCC GGG CTT GCC 48

TCT GGA GCC TCT 60

(18) INFORMATION FOR SEQ ID NO:17:

(i) SEQUENCE CHARACTERISTICS:

(A) LENGTH: 18 base pairs

(B) TYPE: nucleic acid

(C) STRANDEDNESS: single stranded

(D) TOPOLOGY: linear

(xi) SEQUENCE DESCRIPTION: SEQ ID NO:17:

YTT RCA CAT RTA YTC NCC 18

The features disclosed in the foregoing description, in the following claims and/or in the accompanying drawings may, both separately and in any combination thereof, be material for realising the invention in diverse forms thereof.

## Claims

1. A non-naturally-occurring polypeptide comprising an amino acid sequence sufficiently duplicative of that of naturally-occuring *neu* receptor stimulating factor to allow possession of one or more biological activities of naturally-occurring *neu* receptor stimulating factor.

2. A polypeptide according to Claim 1 which is a product of procaryotic or eucaryotic expression of an exogenous DNA sequence.

3. A polypeptide according to Claim 2 which is a product of non-human mammalian cell expression.

4. A polypeptide according to Claim 3 in which the non-human mammalian cell is a CHO cell.

5. A polypeptide according to Claim 2 which is a product of *E. coli* cell expression.

6. A polypeptide according to Claim 2 which is a product of yeast cell expression.

7. A polypeptide according to Claim 2 wherein the exogenous DNA sequence is a cDNA sequence.

8. A polypeptide according to Claim 7 wherein the cDNA sequence is the DNA sequence of Figure 5 (SEQ ID NO:4).

9. A polypeptide according to Claim 2 wherein the exogenous DNA sequence is a genomic DNA sequence which is homologous to the cDNA sequence of Figure 5 (SEQ ID NO:4).

10. A polypeptide according to Claim 2 wherein the exogenous DNA sequence is a manufactured DNA sequence.

11. A polypeptide according to Claim 2 wherein the exogenous DNA sequence is carried on an autonomously replicating DNA plasmid or viral vector.

12. A polypeptide according to claim 1 comprising the amino acid sequence of rat *neu* receptor stimulating factor set forth in Figure 5 (SEQ ID NO:4), or any genetically engineered variant thereof.

13. A polypeptide according to Claim 1 comprising the amino acid sequence of the EGF-like domain of rat *neu* receptor stimulating factor set forth in Figure 7 (SEQ ID NO:5).

14. A polypeptide according to Claim 1 comprising the amino acid sequence of the immunoglobulin-like domain of rat *neu* receptor stimulating factor set forth in Figure 8 (SEQ ID NO:14).

15. A polypeptide according to Claim 1 which has one or more *in vivo* biological activities of naturally-occurring neu receptor stimulating factor.

16. A polypeptide according to Claim 1 which has one or more *in vitro* biological activities of naturally-occurring neu receptor stimulating factor.

17. An isolated DNA sequence for use in securing expression in a procaryotic or eucaryotic host cell of a polypeptide product having an amino acid sequence sufficiently duplicative of that of naturally-occurring *neu* receptor stimulating factor to allow possession of one or more biological activities of naturally occurring *neu* receptor stimulating factor, said DNA sequence selected from among:
(a) the DNA sequences set out in Figures 4 and 5 (SEQ ID NO:3 and SEQ ID NO:4, respectively) or their complementary strands;
(b) DNA sequences which hybridize to the DNA sequences defined in (a) or fragments thereof; and
(c) DNA sequences which, but for the degeneracy of the genetic code, would hybridize to the DNA sequences defined in (a) and (b).

18. A procaryotic or eucaryotic host cell transformed or transfected with a DNA sequence according to Claim 17 in a manner allowing the host cell to express the polypeptide product.

19. A polypeptide product of the expression of a DNA sequence of Claim 17 in a procaryotic or eucaryotic host cell.

20. An isolated DNA sequence encoding procaryotic or eucaryotic host expression of a polypeptide having an amino acid sequence sufficiently duplicative of that of naturally-occurring *neu* receptor stimulating factor to allow possession of one or more biological activities of naturally-occurring *neu* receptor stimulating factor.

21. A cDNA sequence according to Claim 20.

**22.** A genomic DNA sequence according to Claim 20.

**23.** A manufactured DNA sequence according to Claim 20.

**24.** A DNA sequence according to Claim 20 as set out in Figures 4 and 5 (SEQ ID NO:3 and SEQ ID NO:4, respectively).

**25.** A DNA sequence according to Claim 20 including one or more codons preferred for expression in *E.coli* cells.

**26.** A DNA sequence according to Claim 20 including one or more codons preferred for expression in yeast cells.

**27.** A DNA sequence according to Claim 20 including one or more codons preferred for expression in mammalian cells.

**28.** A biologically functional plasmid or viral DNA vector including a DNA sequence according to Claim 20.

**29.** A procaryotic or eucaryotic host cell stably transformed or transfected with a DNA vector according to Claim 20.

**30.** A polypeptide product of the expression in a procaryotic or eucaryotic host cell of a DNA sequence according to Claim 20.

**31.** A DNA sequence encoding a polypeptide fragment or polypeptide analog of naturally-occurring *neu* receptor stimulating factor.

**32.** A DNA sequence as in Claim 31 encoding methionyl *neu* receptor stimulating factor.

**33.** A polypeptide having part or all of the amino acid sequence set forth in Figure 5 (SEQ ID NO:4) and having one or more of the *in vitro* or *in vivo* biological activities of naturally-occurring *neu* receptor stimulating factor.

**34.** A polypeptide having part or all of the secondary structure of naturally-occurring *neu* receptor stimulating factor and having part or all of the amino acid sequence set forth in Figure 5 (SEQ ID NO:4), and having one or more biological properties of naturally-occurring neu receptor stimulating factor.

**35.** A DNA sequence coding for an analog of human neu receptor stimulating factor selected from the group consisting of:
a) [Met-1] *neu* receptor stimulating factor; and
b) *neu* receptor stimulating factor wherein one or more cysteines are replaced by alanine or serine.

**36.** A polypeptide product of the expression in a procaryotic or eucaryotic host cell of a DNA sequence according to Claim 35.

**37.** A non-naturally-occurring polypeptide having one or more biological activities of naturally-occurring *neu* receptor stimulating factor, said polypeptide having an amino acid sequence set forth in Figure 5 (SEQ ID NO:4), or any derivatives, deletion analogs, substitution analogs, or addition analogs thereof, and characterized by being the product of procaryotic or eucaryotic expression of an exogenous DNA sequence.

**38.** A process for the production of *neu* receptor stimulating factor comprising:
growing, under suitable nutrient conditions, procaryotic or eucaryotic host cells transformed or transfected with a DNA according to Claim 17, and
isolating the desired polypeptide product of the expression of the DNA sequence in said vector.

**39.** A method of modulating cellular proliferation and differentiation, comprising contacting the cells with an effective amount of recombinant *neu* receptor stimulating factor.

**40.** A method according to Claim 39 which is carried out in a mammal.

**41.** A method according to Claim 40 in which the mammal is a human.

**42.** A method for enhancing repair and regeneration of human tissues that express the *neu* receptor, comprising administering an effective amount of recombinant *neu* receptor stimulating factor.

**43.** A method according to Claim 42, in which the tissues are selected from the group consisting of the gastrointestinal, respiratory, urinary and reproductive tract tissues.

**44.** A method according to Claim 42, in which the tissues comprise human skin.

**45.** A method according to Claim 42, in which the tissues are neural tissues.

**46.** A method of treating *neu* receptor stimulating factor deficiencies in human tissues that express the *neu* receptor, comprising administering an effective amount of recombinant *neu* receptor stimulating factor to a human having such a deficiency.

**47.** A method of treating a mammalian tumor expressing *neu* receptor on the surface of the tumor cells, comprising administering to a mammal having such a tumor an amount of recombinant *neu* receptor stimulating factor effective to reduce tumor growth.

**48.** A method according to Claim 47, wherein the mammal is a human.

**49.** A method according to Claim 48, used for the treatment of carcinoma selected from the group consisting of prostate, ovary, breast, stomach, lung, kidney and skin carcinomas.

**50.** A pharmaceutical composition comprising an effective amount of recombinant *neu* receptor stimulating factor and a pharmaceutically-acceptable diluent, adjuvant or carrier.

**51.** An antibody specifically generated by immunization with a polypeptide according to Claim 12.

**52.** An antibody according to Claim 51 which is a monoclonal antibody.

**53.** A biologically active composition comprising the polypeptide of Claim 1 covalently attached to a water soluble polymer.

**54.** A composition according to Claim 53 in which the polymer is selected from the group consisting of polyethylene glycol and copolymers of polyethylene glycol and polypropylene glycol.

**55.** A method of detecting underexpression or overexpression of *neu* receptor stimulating factor mRNA in human cells or tissues, comprising:

(a) isolating RNA from the cells or tissues;

(b) contacting the RNA with a nucleic acid probe capable of hybridizing with a nucleic acid sequence present in *neu* receptor stimulating factor mRNA under conditions appropriate for hybridization of the probe with the nucleic acid for which it is specific; and

(c) determining the level of hybridization between the RNA and the probe, as indicative of underexpression or overexpression of the *neu* receptor stimulating factor mRNA.

FIG. I

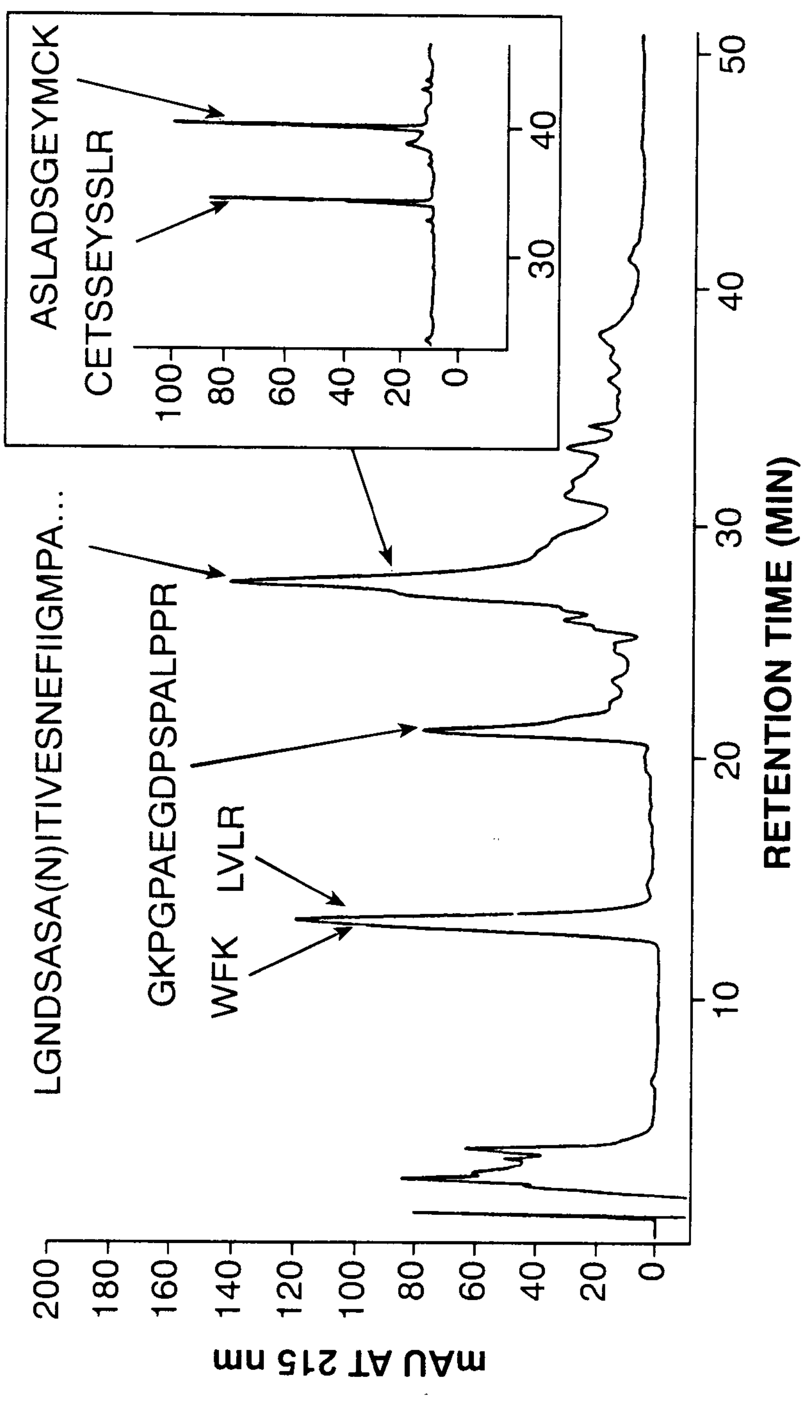
LGNDSASA(N)ITIVESNEFIIGMPA....
GKPGPAEGDPSPALPPR
WFK
LVLR
ASLADSGEYMCK
CETSSEYSSLR
100
80
60
40
20
0
30
40
200
180
160
140
120
100
80
60
40
20
0
mAU AT 215 nm
10
20
30
40
50
RETENTION TIME (MIN)

FIG. 2

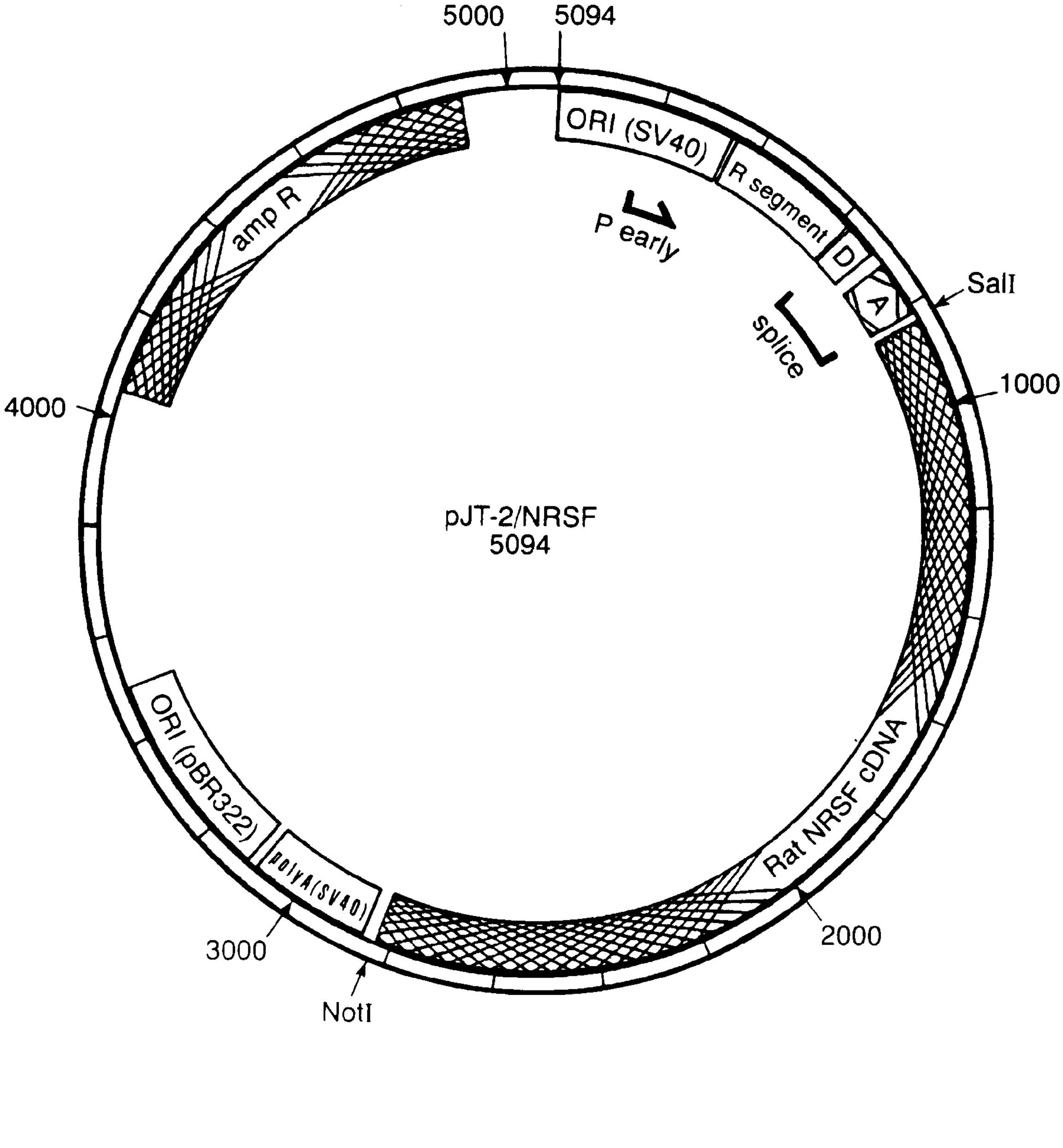
5000
5094
ORI (SV40)
R segment
P early
D
A
SalI
splice
1000
4000
amp R
pJT-2/NRSF
5094
ORI (pBR322)
polyA(SV40)
Rat NRSF cDNA
2000
3000
NotI

FIG. 3

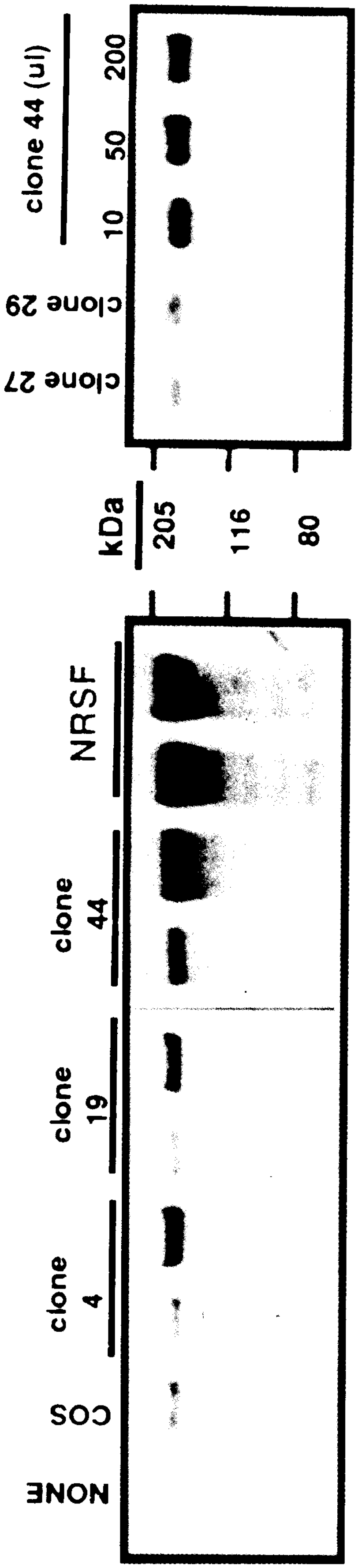

clone 27
clone 29
clone 44 (ul)
10
50
200
kDa
205
116
80
NONE
COS
clone 4
clone 19
clone 44
NRSF

# FIG. 4A

```
  1 AGCTGCCGGGAGATGCGAGCGCAGACCGGATTGTGATCACCTTTCCCTCTTCGGGCTGTAAGAGAGCGAGACAAGCCACCGAAGCGAGGC

 91 CACTCCAGAGCCGGCAGCGGAGGGACCCGGGACACTAGAGCAGCTCCGAGCCACTCCAGACTGAGCGGACGCTCCAGGTGATCGAGTCCA

181 CGCTGCTTCCTGCAGGCGACAGGCGACGCCTCCCGAGCAGCCCGGCCACTGGCTCTTCCCCTCCTGGGACAAACTTTTCTGCAAGCCCTT

                                                                      MetSerGluArgLysGluGlyArg    8
271 GGACCAAACTTGTCGCGCGTCACCGTCACCCAACCGGGTCCGCGTAGAGCGCTCATCTTCGGCGAGATGTCTGAGCGCAAAGAAGGCAGA

    GlyLysGlyLysGlyLysLysLysAspArgGlySerArgGlyLysProGlyProAlaGluGlyAspProSerProAlaLeuProProArg   38
361 GGCAAGGGGAAGGGCAAGAAGAAGGACCGGGGATCCCGCGGGAAGCCCGGGCCCGCCGAGGGCGACCCGAGCCCAGCACTGCCTCCCAGA

    LeuLysGluMetLysSerGlnGluSerAlaAlaGlySerLysLeuValLeuArgCysGluThrSerSerGluTyrSerSerLeuArgPhe   68
451 TTGAAAGAAATGAAGAGCCAGGAGTCAGCTGCAGGCTCCAAGCTAGTGCTCCGGTGCGAAACCAGCTCCGAGTACTCCTCACTCAGATTC

    LysTrpPheLysAsnGlyAsnGluLeuAsnArgLysAsnLysProGluAsnIleLysIleGlnLysLysProGlyLysSerGluLeuArg   98
541 AAATGGTTCAAGAATGGGAACGAGCTGAACCGCAAAAATAAACCAGAAAACATCAAGATACAGAAGAAGCCAGGGAAGTCAGAGCTTCGA

                                                                       *              *
    IleAsnLysAlaSerLeuAlaAspSerGlyGluTyrMetCysLysValIleSerLysLeuGlyAsnAspSerAlaSerAlaAsnIleThr  128
631 ATTAACAAAGCATCCCTGGCTGACTCTGGAGAGTATATGTGCAAAGTGATCAGCAAGTTAGGAAATGACAGTGCCTCTGCCAACATCACC

    IleValGluSerAsnGluPheIleThrGlyMetProAlaSerThrGluThrAlaTyrValSerSerGluSerProIleArgIleSerVal  158
721 ATTGTTGAGTCAAACGAGTTCATCACTGGCATGCCAGCCTCGACTGAGACAGCCTATGTGTCCTCAGAGTCTCCCATTAGAATCTCAGTT

                       *
    SerThrGluGlyAlaAsnThrSerSerSerThrSerThrSerThrThrGlyThrSerHisLeuIleLysCysAlaGluLysGluLysThr  188
811 TCAACAGAAGGCGCAAACACTTCTTCATCCACATCAACATCCACGACTGGGACCAGCCATCTCATAAAGTGTGCGGAGAAGGAGAAAACT

    PheCysValAsnGlyGlyGluCysPheThrValLysAspLeuSerAsnProSerArgTyrLeuCysLysCysGlnProGlyPheThrGly  218
901 TTCTGTGTGAATGGGGGCGAGTGCTTCACGGTGAAGGACCTGTCAAACCCGTCAAGATACTTGTGCAAGTGCCAACCTGGATTCACTGGA

    AlaArgCysThrGluAsnValProMetLysValGlnThrGlnGluLysAlaGluGluLeuTyrGlnLysArgValLeuThrIleThrGly  248
991 GCAAGATGTACTGAGAATGTACCCATGAAAGTCCAAACCCAAGAAAAAGCGGAGGAACTCTACCAGAAGAGGGTGCTGACAATTACTGGC
```

Ig DOMAIN (residues 68–128)

EGF DOMAIN (residues 188–248)

FIG. 4B

IleCysIleAlaLeuLeuValValGlyIleMetCysValValAlaTyrCysLysThrLysLysGlnArgGlnLysLeuHisAspArgLeu 278
1081 ATCTGTATCGCCCTGCTGGTGGTCGGCATCATGTGTGTGGTGGCCTACTGCAAAACCAAGAAGCAGCGGCAGAAGCTTCATGATCGGCTT

ArgGlnSerLeuArgSerGluArgSerAsnLeuValAsnIleAlaAsnGlyProHisHisProAsnProProProGluAsnValGlnLeu 308
1171 CGGCAGAGTCTTCGGTCAGAACGGAGCAACCTGGTGAACATAGCGAATGGGCCTCACCACCCAAACCCACCGCCAGAGAACGTGCAGCTG

ValAsnGlnTyrValSerLysAsnValIleSerSerGluHisIleValGluArgGluValGluThrSerPheSerThrSerHisTyrThr 338
1261 GTGAATCAATACGTATCTAAAAACGTCATCTCCAGTGAGCATATTGTTGAGAGAGAAGTGGAGACTTCCTTTTCCACCAGTCATTACACT

SerThrAlaHisHisSerThrThrValThrGlnThrProSerHisSerTrpSerAsnGlyHisThrGluSerValIleSerGluSerAsn 368
1351 TCCACAGCCCATCACTCCACGACTGTCACCCAGACTCCTAGTCACAGCTGGAGTAATGGGCACACGGAGAGCGTCATTTCAGAAAGCAAC

SerValIleMetMetSerSerValGluAsnSerArgHisSerSerProAlaGlyGlyProArgGlyArgLeuHisGlyLeuGlyGlyPro 398
1441 TCCGTAATCATGATGTCTTCGGTAGAGAACAGCAGGCACAGCAGTCCCGCCGGGGGCCCACGAGGACGTCTTCATGGCCTGGGAGGCCCT

ArgAspAsnSerPheLeuArgHisAlaArgGluThrProAspSerTyrArgAspSerProHisSerGluArg 422
1531 CGTGATAACAGCTTCCTCAGGCATGCCAGAGAAACCCCTGACTCCTACAGAGACTCTCCTCATAGCGAAAGGTAAAATGGAAGGGTAAAG

1621 CTATCGTGGAGGAGAACCTCATTCAGTGAGAGAATCCCATGAGCACCTGCGGTCTCTCCTAAGGAAACTCATCCTTTCATAAGGGGCGTC

1711 ATCAATTCCCTGACGCTCCTAGTTGATGAAGTCAACCTCTTATTTGTCTGAACTCCTTTCTCCTGAGCTTCTCCCCGCGTCCCAGTGGCT

1801 GACAGGCAACCAACTCCTAAAGAGCAGGAGTAATGTAATGTGGAAGGCCCAGCCGACTTGGAGTTCTCAGACCTGACCCTAAGGTCAGAC

1891 TCACTGGGGTCTTCCTTTCGTCAGGTGCACCATTTTAAGGACCCTCATCTAATCCCGATCAGCCAGTTGTCCATTCTCTACCTCGATGGT

1981 TGTTCTGCCCTCCTCTCCTCATGCTCTAAGTACCCAGCCTCTAGCCTCAGTTAAATCAAGTCGAGGGCTGGGACACGGCTGATGGTCATG

2071 GCGGAATGCCTCCTCGGGTTCCACCACACTGTATCCATTTACGACATCCCTAGAAGTCATGGGTAAATAAAGTTGTTGTGTGATGTGAAA

2161 AAAAAAAAAAAAAAAAAAAAAAAAAA

# FIG. 5A

```
                                             MetSerGluArgLysG
CCGTCACCCAACCGGGTCCGCGTAGAGCGCTCATCTTCGGCGAGATGTCTGAGCGCAAAG   60

luGlyArgGlyLysGlyLysGlyLysLysLysAspArgGlySerArgGlyLysProGlyP
AAGGCAGAGGCAAGGGGAAGGGCAAGAAGAAGGACCGGGGATCCCGCGGGAAGCCCGGGC  120

roAlaGluGlyAspProSerProAlaLeuProProArgLeuLysGluMetLysSerGlnG
CCGCCGAGGGCGACCCGAGCCCAGCACTGCCTCCCAGATTGAAAGAAATGAAGAGCCAGG  180

luSerAlaAlaGlySerLysLeuValLeuArgCysGluThrSerSerGluTyrSerSerL
AGTCAGCTGCAGGCTCCAAGCTAGTGCTCCGGTGCGAAACCAGCTCCGAGTACTCCTCAC  240

euArgPheLysTrpPheLysAsnGlyAsnGluLeuAsnArgLysAsnLysProGluAsnI
TCAGATTCAAATGGTTCAAGAATGGGAACGAGCTGAACCGCAAAAATAAACCAGAAAACA  300

leLysIleGlnLysLysProGlyLysSerGluLeuArgIleAsnLysAlaSerLeuAlaA
TCAAGATACAGAAGAAGCCAGGGAAGTCAGAGCTTCGAATTAACAAAGCATCCCTGGCTG  360

spSerGlyGluTyrMetCysLysValIleSerLysLeuGlyAsnAspSerAlaSerAlaA
ACTCTGGAGAGTATATGTGCAAAGTGATCAGCAAGTTAGGAAATGACAGTGCCTCTGCCA  420

snIleThrIleValGluSerAsnGluPheIleThrGlyMetProAlaSerThrGluThrA
ACATCACCATTGTTGAGTCAAACGAGTTCATCACTGGCATGCCAGCCTCGACTGAGACAG  480

laTyrValSerSerGluSerProIleArgIleSerValSerThrGluGlyAlaAsnThrS
CCTATGTGTCCTCAGAGTCTCCCATTAGAATCTCAGTTTCAACAGAAGGCGCAAACACTT  540
```

FIG. 5B

```
erSerSerThrSerThrSerThrThrGlyThrSerHisLeuIleLysCysAlaGluLysG
CTTCATCCACATCAACATCCACGACTGGGACCAGCCATCTCATAAAGTGTGCGGAGAAGG    600

luLysThrPheCysValAsnGlyGlyGluCysPheThrValLysAspLeuSerAsnProS
AGAAAACTTTCTGTGTGAATGGGGGCGAGTGCTTCACGGTGAAGGACCTGTCAAACCCGT    660

erArgTyrLeuCysLysCysGlnProGlyPheThrGlyAlaArgCysThrGluAsnValP
CAAGATACTTGTGCAAGTGCCAACCTGGATTCACTGGAGCAAGATGTACTGAGAATGTAC    720

roMetLysValGlnThrGlnGluLysAlaGluGluLeuTyrGlnLysArgValLeuThrI
CCATGAAAGTCCAAACCCAAGAAAAAGCGGAGGAACTCTACCAGAAGAGGGTGCTGACAA    780

leThrGlyIleCysIleAlaLeuLeuValValGlyIleMetCysValValAlaTyrCysL
TTACTGGCATCTGTATCGCCCTGCTGGTGGTCGGCATCATGTGTGTGGTGGCCTACTGCA    840

ysThrLysLysGlnArgGlnLysLeuHisAspArgLeuArgGlnSerLeuArgSerGluA
AAACCAAGAAGCAGCGGCAGAAGCTTCATGATCGGCTTCGGCAGAGTCTTCGGTCAGAAC    900

rgSerAsnLeuValAsnIleAlaAsnGlyProHisHisProAsnProProProGluAsnV
GGAGCAACCTGGTGAACATAGCGAATGGGCCTCACCACCCAAACCCACCGCCAGAGAACG    960

alGlnLeuValAsnGlnTyrValSerLysAsnValIleSerSerGluHisIleValGluA
TGCAGCTGGTGAATCAATACGTATCTAAAAACGTCATCTCCAGTGAGCATATTGTTGAGA    1020

rgGluValGluThrSerPheSerThrSerHisTyrThrSerThrAlaHisHisSerThrT
GAGAAGTGGAGACTTCCTTTTCCACCAGTCATTACACTTCCACAGCCCATCACTCCACGA    1080

hrValThrGlnThrProSerHisSerTrpSerAsnGlyHisThrGluSerValIleSerG
CTGTCACCCAGACTCCTAGTCACAGCTGGAGTAATGGGCACACGGAGAGCGTCATTTCAG    1140
```

# FIG. 5C

```
luSerAsnSerValIleMetMetSerSerValGluAsnSerArgHisSerSerProAlaG
AAAGCAACTCCGTAATCATGATGTCTTCGGTAGAGAACAGCAGGCACAGCAGTCCCGCCG   1200

lyGlyProArgGlyArgLeuHisGlyLeuGlyGlyProArgAspAsnSerPheLeuArgH
GGGGCCCACGAGGACGTCTTCATGGCCTGGGAGGCCCTCGTGATAACAGCTTCCTCAGGC   1260

isAlaArgGluThrProAspSerTyrArgAspSerProHisSerGluArg
ATGCCAGAGAAACCCCTGACTCCTACAGAGACTCTCCTCATAGCGAAAGGTAAAATGGAA   1320

GGGTAAAGCTATCGTGGAGGAGAACCTCATTCAGTGAGAGAATCCCATGAGCACCTGCGG   1380

TCTCTCCTAAGGAAACTCATCCTTTCATAAGGGGCGTCATCAATTCCCTGACGCTCCTAG   1440

TTGATGAAGTCAACCTCTTATTTGTCTGAACTCCTTTCTCCTGAGCTTCTCCCCGCGTCC   1500

CAGTGGCTGACAGGCAACCAACTCCTAAAGAGCAGGAGTAATGTAATGTGGAAGGCCCAG   1560

CCGACTTGGAGTTCTCAGACCTGACCCTAAGGTCAGACTCACTGGGGTCTTCCTTTCGTC   1620

AGGTGCACCATTTTAAGGACCCTCATCTAATCCCGATCAGCCAGTTGTCCATTCTCTACC   1680

TCGATGGTTGTTCTGCCCTCCTCTCCTCATGCTCTAAGTACCCAGCCTCTAGCCTCAGTT   1740

AAATCAAGTCGAGGGCTGGGACACGGCTGATGGTCATGGCGGAATGCCTCCTCGGGTTCC   1800

ACCACACTGTATCCATTTACGACATCCCTAGAAGTCATGGGTAAATAAAGTTGTTGTGTG   1860

ATGTGAAAAAAAAAAAAAAAAAAAAAAAAAAAAA   1894
```

FIG. 6

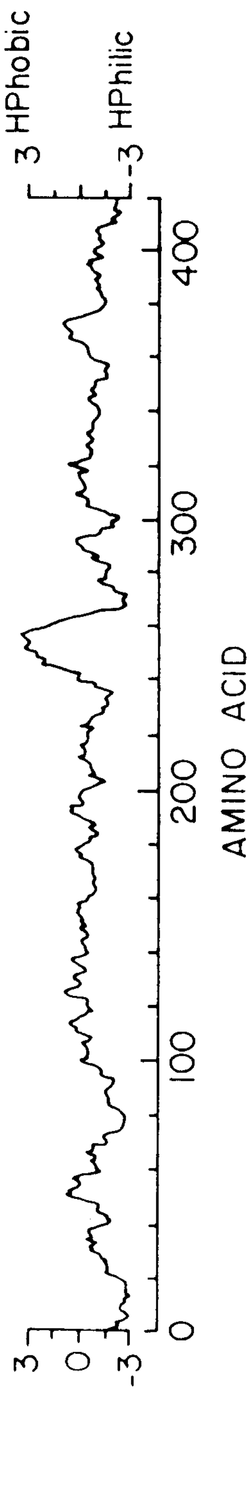

FIG. 7

```
                 1                  10                  20                  30                  40                  50                  60
NRSF (rat)       C A E K E K T F C V N G G E C F T V K D L S N P S R Y L C K C Q P G F T G A R C T E N V P M K V Q T Q E K A E E L Y Q K R V L T
TGFα (rat)       C P D S H T Q Y C F H - G T C R F L V Q E E K P A - - - C V C H S G Y V G V R C E H A D L L A V V A A S Q K K Q A I T A A V V V
AR (human)       C N A E F Q N F C I H - G E C K Y I E H L E A V T - - - C K C Q Q E Y F G E R C G E K S M K T H S M I D S S L S K I A L L A I A
EGF (mouse)      C P S S Y D G Y C L N G G V C M H I E S L D S Y T - - - C N C V I G Y S G D R C Q T R D L R W W E L R H A G Y G Q K H D I M V V
HB-EGF (human)   C L R K Y K D F C I H - G E C K Y V K E L R A P S - - - C I C H P G Y H G E R C H G L S L P V E N R L Y T Y D H T T I L A V V A
SDGF (rat)       C A A K F Q N F C I H - G E C R Y I E N L E V V T - - - C H C H Q D Y F G E R C G E K T M K T Q K K D D S D L S K I A L A A I I
VGF (viral)      C G P E G D G Y C L H - G D C I H A R D I D G M Y - - - C R C S H G Y T G I R C Q H V V L V D Y Q R S E N P N T T T S Y I P S P
SFGF (viral)     C N H D Y E N Y C L N N G T C F T I A L D N V S I T P F C V C R I N Y E G S R C Q F I N L V T Y *
MGF (viral)      C N D D Y K N Y C L N N G T C F T V A L N N V S L N P F C A C H I N Y V G S R C Q F I N L I T I K *
```

FIG. 8

B

```
NRSF (rat)     LVLRCETSSEYSSLRFKWFKNGNELNRKNNKPENIKIQKKPGK-SELRINKASLADSGEYMCKVIS
NCAM (mouse)   VTLTCEASGDPIPSIT-WRTSTRNISSEEQDLDGHMVVRSHARVSSLTLKSIQYRDAGEYMCTASN

C-2 SET        VTLTCEA  NP   L W                     LL  VT  D G Y C A N
               β-STRAND  B      C          D          E              F
```

FIG. 9

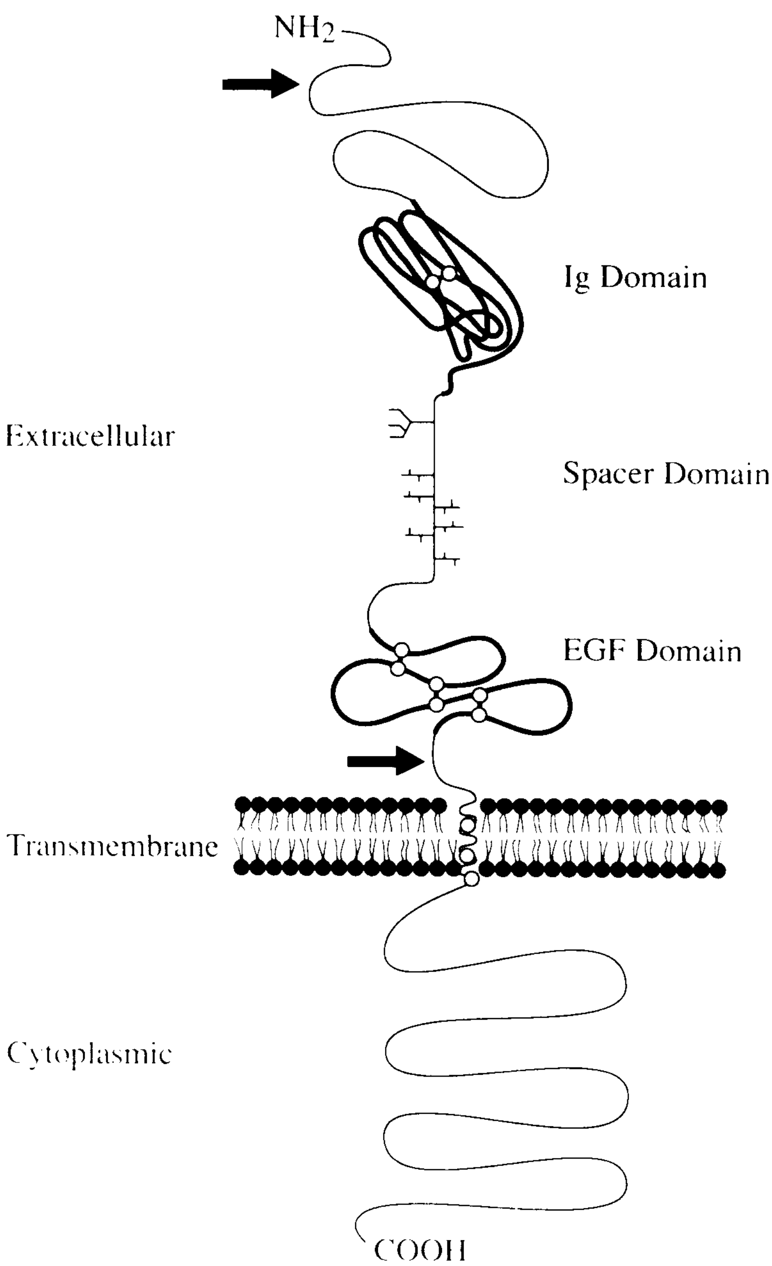
NH2
Ig Domain
Extracellular
Spacer Domain
EGF Domain
Transmembrane
Cytoplasmic
COOH

FIG. 10

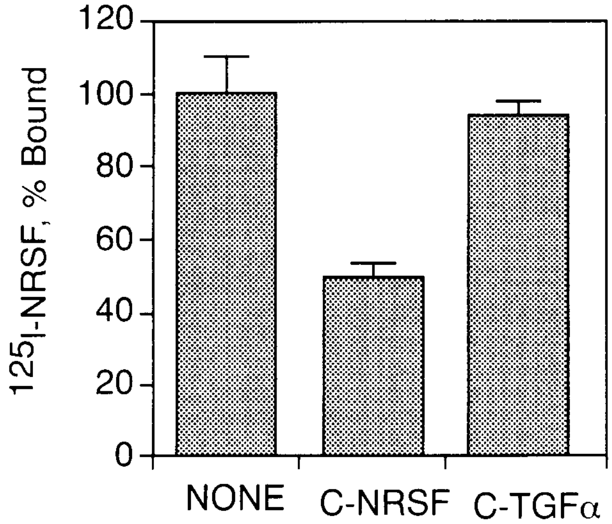
120
100
80
60
40
20
0
$^{125}$I-NRSF, % Bound
NONE
C-NRSF
C-TGFα

FIG. 11

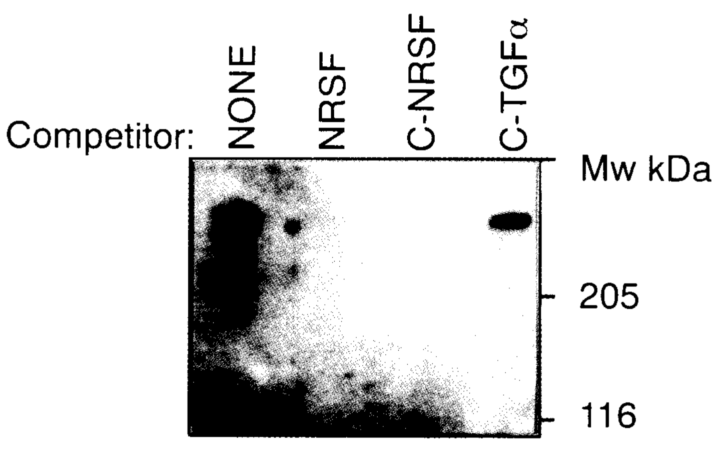
Competitor:
NONE
NRSF
C-NRSF
C-TGFα
Mw kDa
205
116

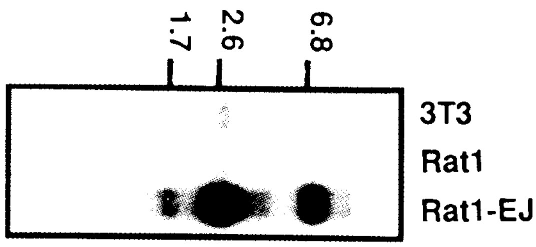


FIG. 12A

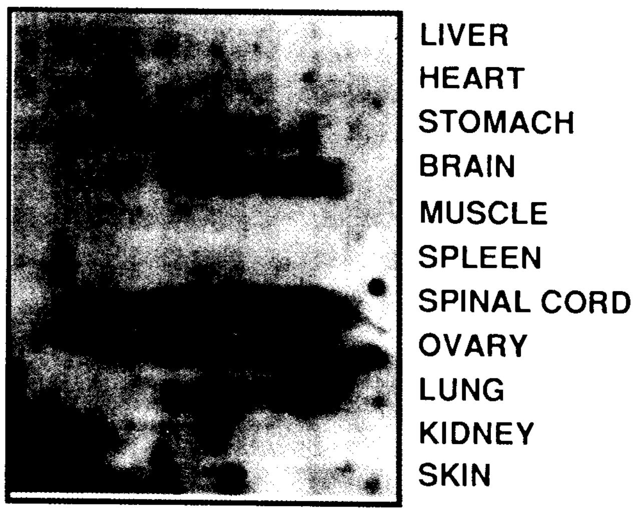


FIG. 12B

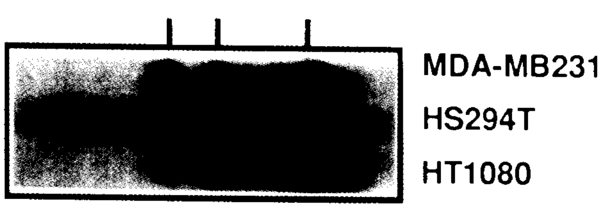


FIG. 12C